# FASCICULE DE BREVET EUROPEEN

(11) **EP 0 569 272 B1**
(45) Date de publication et mention de la délivrance du brevet: **17.06.1998**
(21) Numéro de dépôt: 93401119.8
(22) Date de dépôt: 29.04.1993
(51) Int. Cl.: C12Q 1/68

(54) **Procédé d'amplification d'ARN nécessitant une seule étape de manipulation**
Einstufiges Amplifikationsverfahren für RNS
Single-step amplification method for RNA

(30) Priorité: 29.04.1992 FR 9205322
(43) Date de publication de la demande: 10.11.1993
(73) Titulaire: BIO MERIEUX, 69280 Marcy l'Etoile (FR)
(72) Inventeur: Mallet, François, F-69100 Villeurbanne (FR); Oriol, Guy, F-42400 Saint-Chamond (FR); Mandrand, Bernard, F-69100 Villeurbanne (FR)
(74) Mandataire: Tonnellier, Jean-Claude

(56) Documents cités:
- EP-A- 0 397 463
- EP-A- 0 479 158
- WO-A-86/01827
- WO-A-91/09944
- FR-A- 2 669 347
- US-A- 5 021 335
- GENE vol. 88, no. 2, 16 Avril 1990, AMSTERDAM NL pages 293 - 296 W.T.TSE ET AL. 'Reverse transcription and direct amplification of cellular RNA transcripts by Taq polymerase'
- GENE ANALYSIS TECHNIQUES vol. 6, no. 2, Mars 1989, NEW YORK US pages 25 - 28 E. SHIMOMAYE ET AL. 'Use of avian myeloblastosis virus reverse transcriptase at high temperature for sequence analysis of highly structured RNA'

## Description

L'invention a pour objet un procédé d'amplification d'acide ribonucléique (ARN), dans lequel tous les ingrédients nécessaires aux diverses réactions sont introduits dans la solution de départ, l'ensemble des étapes étant effectué dans un même conteneur et sans ajout ultérieur d'aucun ingrédient. On peut ainsi opérer dans un conteneur fermé pendant toute la durée des opérations, et donc réduire les risques de contamination accidentelle et les risques d'erreur de manipulation.

On sait actuellement amplifier une séquence d'ADN, sans qu'il soit nécessaire de la cloner dans une cellule-hôte. La technique la plus connue dans ce domaine est celle dite d'amplification en chaîne par polymérase, souvent désignée par le sigle PCR, décrite par exemple dans les brevets US 4.683.195 et 4.683.202.

La méthode d'amplification PCR peut être combinée à une étape préalable de transcription réverse pour amplifier, sous la forme d'ADN, une séquence d'ARN.

La réaction d'amplification en chaîne par polymérase couplée à la transcription réverse (souvent désignée en abrégé par le sigle RT-PCR) est maintenant largement utilisée pour suivre l'expression des gènes au niveau des ARN messagers (ARNm) ainsi que pour le clonage direct de séquences codantes.

La réaction d'amplification en chaîne par polymérase couplée à la transcription réverse consiste donc à réaliser la synthèse d'un premier brin d'ADN complémentaire (ADNc) à l'aide d'une transcriptase réverse, à dénaturer l'hétéroduplex ARN-ADNc formé, à synthétiser un second brin d'ADNc, complémentaire du premier brin, sous l'action d'une ADN polymérase, puis à soumettre l'ADNc double brin obtenu à une amplification en chaîne par polymérase, selon les méthodes connues, qui consistent en une série de réactions successives de dénaturation de l'ADN double-brin suivie de sa réplication sous l'action d'une ADN polymérase.

Plus précisément, la synthèse du premier brin d'ADNc est réalisée à l'aide de nucléosides triphosphates, par élongation d'une amorce oligonucléotidique sous l'action d'une transcriptase réverse. La copie de la matrice d'ARN étant effectuée dans le sens 3' → 5' de la matrice, l'amorce oligonucléotidique doit être capable de s'hybrider avec une séquence située au voisinage de l'extrémité 3' de l'ARN à copier. Pour cette raison, l'amorce utilisée lors de l'opération de transcription réverse est appelée amorce 3'.

Pour réaliser la synthèse du second brin d'ADN complémentaire, on procède par synthèse du produit d'élongation d'une seconde amorce capable de s'hybrider avec une séquence voisine de l'extrémité 3' du premier brin d'ADNc. Autrement dit, la seconde amorce doit être identique à une séquence voisine de l'extrémité 5' de l'ARN de départ, ou doit être suffisamment homologue d'une telle séquence pour pouvoir s'hybrider avec ladite séquence du premier brin d'ADNc. Pour cette raison, cette seconde amorce est appelée amorce 5'.

Les deux amorces encadrent donc la séquence à amplifier.

La réaction de RT-PCR comporte, indépendamment de la méthodologie utilisée, plusieurs étapes réactionnelles successives, à savoir la dénaturation de l'ARN, l'hybridation de l'amorce 3' sur l'ARN, la synthèse d'un premier brin d'ADNc à l'aide d'une enzyme présentant une activité ADN polymérase ARN-dépendante (ou transcriptase réverse), la dénaturation de l'hétéroduplex ARN-ADNc formé, l'hybridation de l'amorce 5' sur le premier brin d'ADNc, la synthèse du second brin d'ADNc à l'aide d'une enzyme présentant une activité ADN polymérase ADN-dépendante, puis la succession des réactions d'amplification par dénaturation de l'ADN double-brin, hybridation des amorces 3' et 5' avec le brin dont elles sont respectivement complémentaires et synthèse des seconds brins d'ADN par élongation à partir des amorces.

La méthode RT-PCR est très sensible. Cette grande sensibilité est d'ailleurs souvent à l'origine de résultats "faux-positifs" provoqués par des traces résiduelles d'ADN génomique dans les préparations d'ARNm. Pour des raisons analogues, il est très important d'éviter l'introduction accidentelle de séquences contaminantes. Il est donc souhaitable de réduire au maximum les risques de contamination pendant la suite de réactions constituant l'amplification en chaîne. Toutefois, suivant la méthodologie utilisée, diverses manipulations peuvent être requises lors de la réaction, accroissant le risque d'introduction de séquences contaminantes.

Dans sa mise en oeuvre la moins élaborée, la méthode RT-PCR comporte trois étapes, à savoir :
1) la dénaturation de l'ARN par chauffage ;
2) la synthèse du premier brin d'ADNc dans un tampon contenant, outre des nucléotides triphosphates, l'amorce 3' et une transcriptase réverse ; et
3) la synthèse du second brin d'ADNc par addition de l'amorce 5' et d'une ADN polymérases, suivie de la suite de réactions d'amplification par PCR ; voir par exemple Stefan SCHWARTZ, Journal of Virology, Vol.64, n°6, Juin 1990, pp.2519-259

Dans le but de simplifier la méthode et de réduire le nombre des manipulations, une méthode simplifiée a été décrite, basée sur la découverte d'un tampon commun pour la transcriptase réverse et pour l'ADN polymérase Taq. Cette méthode simplifiée consiste : 1) à dénaturer l'ARN en solution aqueuse, les deux amorces 3' et 5' étant déjà présentes, et 2) à ajouter à la solution aqueuse les ingrédients du tampon, la transcriptase réverse et l'ADN polymérase. Ensuite, sans ajout ultérieur de réactif, des opérations successives de synthèse du premier brin d'ADNc, de dénaturation du duplex ARN-ADNc, de synthèse du second brin d'ADNc et de cycles d'amplification sont mises en oeuvre aux diverses températures appropriées ; voir notamment C. GOBLET et al., Nucl. Acids Res., 17(5), 2144 (1989). Cette méthode est appelée improprement "méthode en une étape" alors qu'en fait, comme on vient de le voir, elle en comporte deux, puisque la réaction de dénaturation et d'hybridation est effectuée avant l'ajout des ingrédients nécessaires aux étapes de transcription réverse et de synthèse d'ADN double brin, ce qui accroît, tout comme dans la méthode classique en trois étapes, le risque d'introduire des séquences contaminantes.

Une autre technique a été développée consistant à utiliser une enzyme thermostable (rTth) présentant à la fois une activité ADN polymérase ARN-dépendante (transcriptase réverse) et une activité ADN polymérase ADN-dépendante. Toutefois, la première activité nécessite la présence d'ions manganèse tandis que la seconde nécessite la présence d'ions magnésium ; voir MYERS T.W. et al., Biochem. 30, 7661-7666 (1991). Il est donc nécessaire d'effectuer la synthèse du premier brin d'ADNc en présence de MnCl₂, suivie d'une dilution de l'échantillon avec apport de l'amorce 5', de MgCl₂, et d'un agent chélatant qui a le pouvoir de chélater fortement les ions manganèse. Ici encore, un procédé de dilution, avec apport de réactifs, suit nécessairement la synthèse de l'ADNc, ce qui accroît le risque d'introduire des séquences contaminantes.

Il a également été décrit que la Taq polymérase possède une activité ADN-polymérase ARN-dépendante (voir GENE, vol.88, n°2, 16 Avril 1990, Amsterdam NL). Toutefois, il a été montré dans WO91/09944 que cette activité d'ADN-polymérase ARN-dépendante nécessite pour être efficace la présence d'ions manganèse tandis que l'activité ADN-polymérase ADN-dépendante nécessite la présence d'ions magnésium avec de préférence chélatation des ions manganèse.

On a maintenant découvert qu'il est possible d'effectuer l'amplification couplée à la transcription réverse par une méthode ne nécessitant véritablement qu'une seule étape de manipulation, c'est-à-dire une méthode dans laquelle tous les réactifs et solvants sont tous introduits dès le départ dans le conteneur réactionnel, avant l'étape de dénaturation de l'ARN. Cela permet d'opérer de façon simple et en évitant tout risque de contamination lors d'un ajout ultérieur de réactifs ou solvants (on appelle ici solvant tout véhicule liquide ou tout constituant liquide du milieu dans lequel on opère). En outre, on a découvert que, de façon surprenante, cette façon d'opérer en une seule étape est plus efficace que d'opérer selon les techniques antérieures, comme cela sera montré dans la partie expérimentale ci-après.

Les problèmes posés par la réalisation de la RT-PCR avec introduction dès le départ de l'ensemble des réactifs sont liés notamment au choix d'une transcriptase réverse capable de fonctionner après avoir été portée à température relativement élevée pendant l'étape de dénaturation de l'ARN par la chaleur. Cette étape de dénaturation de l'ARN par la chaleur est d'une très grande importance pour la réussite et la fiabilité d'une méthode d'amplification. En outre, la transcriptase réverse doit, après ce traitement par la chaleur, être encore capable de fonctionner à une température relativement élevée. On sait en effet, que pour accroître la spécificité de la réaction d'amplification, il est utile d'opérer dans des conditions suffisamment discriminantes, c'est-à-dire notamment à la température la plus élevée possible, de façon à éviter un appariement non spécifique de l'amorce 3' qui risquerait d'entraîner l'amplification. d'une séquence autre que la séquence cible, et aussi d'opérer à température suffisamment élevée pour éviter la renaturation de l'ARN à amplifier.

Il est également nécessaire que l'ADN polymérase ADN-dépendante résiste à la chaleur, y compris lors de l'étape de dénaturation de l'hétéroduplex ARN-ADNc qui nécessite des températures élevées, généralement supérieures à 90°C. Ce problème est en fait déjà résolu puisqu'on a décrit l'utilisation d'une ADN polymérase provenant de microorganismes capables de survivre dans des conditions de températures élevées, et en particulier la polymérase Taq qui est capable de supporte des températures de l'ordre de 95°C.

On connaît déjà, comme signalé ci-dessus, une transcriptase réverse thermostable et capable de jouer en outre le rôle d'ADN polymérase, mais qui nécessite une modification de l'environnement ionique pour exercer cette deuxième fonction (voir MYERS et al., article cité ci-dessus). Une telle enzyme n'est donc pas utilisable dans une méthode où tous les ingrédients doivent être introduits ensemble dès le départ.

On a maintenant découvert que certaines transcriptases réverses, non réputées thermostables, sont capables, lorsqu'elles sont utilisées en quantité suffisante, de fonctionner à des températures relativement élevées même après avoir subi le traitement thermique nécessaire à l'étape de dénaturation de l'ARN.

Il avait déjà été décrit dans "Gene Analysis Technics", Vol. 16, n°2, pp.25-28 (Mars 1989) dans le cadre d'une étude sur la synthèse d'ADNc à l'aide de la transcriptase réverse du virus de la myéloblastose aviaire que le fait d'élever la température de réaction à 47°C permettait d'éviter la formation de structures secondaires dans la matrice d'ARN tout en conservant une activité de transcription réverse suffisante. Toutefois, dans le procédé décrit dans cet article, la transcriptase réverse est ajoutée après l'étape initiale de dénaturation.

La présente invention a donc pour objet un procédé d'amplification d'au moins une séquence spécifique d'ARN, contenu ou susceptible d'être contenu dans un échantillon, comprenant les étapes consistant à :
- obtenir une solution de départ contenant l'ARN dudit échantillon dans un tampon approprié,
- dénaturer par chauffage l'ARN contenu dans ladite solution,
- traiter la solution obtenue, contenant l'ARN dénaturé, avec une première amorce (ou amorce 3') dans des conditions permettant l'hybridation de l'amorce suivie de la synthèse d'un premier brin d'ADN complémentaire de la séquence d'ARN à amplifier, en présence d'une quantité suffisante d'un système enzymatique ayant, dans ledit tampon, une activité de transcriptase réverse,
- dénaturer l'hétéroduplex ARN-ADNc formé et traiter la solution obtenue avec une seconde amorce (ou amorce 5') dans des conditions permettant l'hybridation de la seconde amorce suivie de la synthèse du second brin d'ADNc, en présence d'un système enzymatique thermostable ayant, dans le même tampon, une activité d'ADN polymérase,
- et à soumettre l'ADNc obtenu à des cycles d'amplification en nombre suffisant pour obtenir le degré d'amplification désiré,
caractérisé par le fait que ladite solution contient dès le départ tous les réactifs et solvants nécessaires auxdites étapes, que l'ensemble des étapes est effectué dans un même conteneur et sans ajout de réactifs ou solvants, et que l'on effectue la dénaturation de l'ARN à une température supérieure ou égale à 60°C environ.

La solution de départ est généralement un tampon aqueux approprié permettant à la transcriptase réverse et à l'ADN polymérase de fonctionner, par exemple un tampon Tris, ayant un pH de 7 à 8,5 et de préférence supérieur à 8. Cette solution de départ contient notamment les ions nécessaires aux activités de transcriptase réverse et d'ADN polymérase, notamment des ions alcalins (Na, K) introduits par exemple sous forme de sels tels qu'un chlorure, à une concentration pouvant aller de 20 à 50 mM, et des ions magnésium, introduits par exemple sous forme de sel de magnésium tel que le chlorure, à une concentration pouvant aller notamment de 1 à 6 mM, par exemple de 1 à 5 mM. Elle peut contenir en outre divers ingrédients usuels, par exemple ceux qui sont utilisés dans la partie expérimentale ci-après.

La solution de départ peut contenir par exemple de 2,5 ng à 1 µg d'ARN total de l'échantillon à amplifier, pour un volume final de 100 µl. L'ARN peut être par exemple dissous dans le milieu liquide ou être introduit dans celui-ci à l'état adsorbé sur un support solide approprié.

La solution de départ contient bien entendu des nucléosides triphosphates en quantité suffisante pour permettre la synthèse de l'ADN complémentaire, de même que la synthèse des brins d'ADN lorsque la méthode d'amplification est du type PCR. Les nucléosides triphosphates peuvent être remplacés, totalement ou partiellement, par des nucléosides modifiés et/ou marqués permettant l'élongation des brins à synthétiser à partir des amorces.

Un point critique dans l'amplification de l'ARN réside dans les structures secondaires de l'ARN qui peuvent induire des terminaisons précoces lors de l'étape de transcription réverse. Pour cette raison, l'amplification débute par une étape de dénaturation de l'ARN qui peut être réalisée par exemple par chauffage à une température généralement supérieure à 60°C, par exemple comprise entre 60 et 75°C, pendant 1 à 15 minutes. Les conditions optimales, qui varient notamment selon la nature de l'ARN, peuvent être déterminées dans chaque cas par de simples expériences de routine. On opère de préférence à une température au moins égale à 65°C.

L'amorce 3' est généralement une amorce d'ADN. Le choix de l'amorce est déterminé par des critères connus. 11 est possible d'utiliser une amorce oligonucléotidique oligo-dT. Pour plus de spécificité, lorsque la séquence du gène est connue, ou lorsque la séquence de la protéine correspondante est connue, on pourra choisir de façon plus précise la localisation de l'amorce 3'. En particulier, les problèmes liés à une contamination éventuelle par de l'ADN peuvent être maîtrisés dans certains cas en sélectionnant des amorces situées dans des exons séparés ou chevauchant des jonctions de sites d'épissage, ce qui induit dans le premier cas la coamplification de l'ADN et de l'ARN, et on obtient alors des produits d'amplification de tailles différentes facilement séparables, et dans le second cas, l'amplification exclusive de l'ARN. Enfin, dans certains cas, on sera amené à choisir des amorces oligonucléotidiques non strictement complémentaires de la cible, pour l'amplification d'ARN de virus endogènes non identifiés ou de séquences inconnues théoriquement proches de séquences connues ; c'est le cas notamment lorsque l'on recherche la présence d'un rétrovirus inconnu : on peut alors utiliser une amorce 3' complémentaire d'une séquence généralement bien conservée chez les rétrovirus, par exemple une séquence de la région "pol".

De préférence, l'amorce 3' a une longueur (en nombre de nucléotides) suffisante pour pouvoir s'hybrider à l'ARN à la température de dénaturation choisie. On sait que la force d'adhésion d'un ADN hybridé à un ARN ou à un ADN dépend de la nature des bases et du nombre de bases appariées. Lorsque le duplex d'hybridation est chauffé, il se produit, à une certaine température, une rupture des liaisons hydrogène entre les bases, avec séparation des deux brins à cette température, dite température de dénaturation ou température de fusion. Cette température de dénaturation augmente avec le nombre de bases, et plus précisément avec le nombre de bases parfaitement appariées. Dans le cas présent, il est généralement souhaitable de pouvoir obtenir une hybridation de l'amorce 3' à la température de dénaturation de l'ARN afin d'éviter une renaturation de l'ARN pendant cette étape d'hybridation. Pour la même raison, on préférera généralement refroidir rapidement, à l'issue de l'étape de dénaturation de l'ARN, jusqu'à la température à laquelle s'effectuera l'étape de transcription réverse.

Généralement, l'amorce 3' contient au moins 15 nucléotides et en particulier de 15 à 30 nucléotides, ou davantage lorqu'il est possible d'opérer à température élevée, le choix de la température élevée dépendant notamment de la capacité de la transcriptase réverse à supporter ladite température.

Il est possible de déterminer par de simples expériences de routine la température et la durée du traitement thermique que la transcriptase réverse est capable de supporter sans perte notable d'activité. On pourra donc effectuer la dénaturation de l'ARN et l'hybridation de l'amorce 3' pendant un temps suffisant pour permettre l'hybridation, mais non supérieur à une durée prédéterminée au-delà de laquelle le système enzymatique à activité de transcriptase réverse risquerait d'être inactivé.

Il convient de noter que l'amorce 3' peut comporter, du côté de son extrémité 5', une queue oligonucléotidique non spécifique qui n'intervient pas dans l'hybridation avec la cible, c'est-à-dire avec l'ARN à amplifier. Cette queue non spécifique peut comporter par exemple de façon connue un site de restriction (qui facilite un clonage ultérieur), une séquence promotrice (qui facilite une transcription ultérieure), ou toute séquence particulière pouvant favoriser un procédé de réplication ultérieur.

Après l'étape de dénaturation et d'hybridation, on peut refroidir la solution, de préférence rapidement, jusqu'à une température prédéterminée permettant la mise en oeuvre de l'activité de transcriptase réverse, ladite température étant suffisamment élevée pour éviter la renaturation de l'ARN, et aussi pour maintenir l'hybridation de l'amorce 3' dans des conditions suffisamment discriminantes, c'est-à-dire suffisamment élevée pour éviter toute hybridation insuffisamment spécifique de l'amorce 3', étant entendu qu'à la température choisie, la transcriptase réverse doit pouvoir exercer son activité.

La température choisie pour l'étape de transcription réverse est de préférence au moins égale à 45 ou à 50°C environ, et par exemple comprise entre 50 et 75°C environ, notamment entre 50 et 65°C, et en particulier entre 50 et 60°C.

Lorsque la séquence d'ARN à amplifier est inconnue ou n'est qu'imparfaitement connue, et que l'on utilise alors une amorce 3' non strictement complémentaire de la cible, il est souhaitable qu'après l'étape de dénaturation de l'ARN, on refroidisse la solution à une température suffisamment basse pour permettre l'hybridation de l'amorce 3' à une séquence d'ARN non rigoureusement complémentaire, pendant un temps suffisamment faible pour éviter toutefois des hybridations purement aléatoires ou non spécifiques, après quoi on réchauffe la solution jusqu'à la température prédéterminée choisie pour l'étape de transcription réverse. Dans ce cas, on refroidit jusqu'à une température inférieure à 50°C, mais au moins égale à 40°C, en procédant par exemple par essais à des températures de plus en plus basses. On peut ensuite remonter la température jusqu'à celle choisie pour l'étape de transcription réverse ; le choix de cette température a déjà été discuté précédemment.

La durée de l'étape de transcription réverse dépend évidemment de la longueur du brin d'ARN à amplifier. On aura intérêt à choisir le temps le plus court, mais suffisant, permettant d'obtenir un brin d'ADNc ayant une longueur suffisante pour contenir au moins la séquence complémentaire de la deuxième amorce (amorce 5'). Lorsque cela sera possible, on déterminera la durée de l'opération de transcription réverse par des expériences de routine. Dans les autres cas, ce temps devra être estimé en prenant garde notamment de ne pas opérer à température trop élevée ou pendant des temps trop longs, de façon à éviter des phénomènes de "fatigue" (perte d'efficacité) de la transcriptase réverse lorsque la séquence d'ARN est relativement longue.

La durée de l'étape de synthèse du premier brin d'ADNc sera généralement inférieure à 15 minutes. De préférence, cette durée peut varier de 1 à 10 minutes environ.

Pour l'opération de transcription réverse, on pourra utiliser une transcriptase réverse non réputée thermostable, choisie après une simple expérimentation permettant de déterminer les traitements thermiques que l'enzyme étudiée est capable de supporter (dans le cadre défini plus haut), ce qui permet de sélectionner les transcriptases réverses qui sont utilisables dans le procédé de l'invention. On a en effet découvert, comme déjà indiqué ci-dessus, que des transcriptases réverses non réputées thermostables l'étaient en fait suffisamment pour pouvoir être utilisées dans un procédé d'amplification d'ARN avec introduction de l'ensemble des réactifs dans la solution de départ.

La transcriptase réverse peut être choisie par exemple parmi les transcriptases réverses de virus de la myéloblastose aviaire (en abrégé RT-AMV), et les transcriptases réverses de virus de Maloney de la leucémie murine (en abrégé RT-MMuLV). On peut citer par exemple la RT-AMVbo (Boehringer-Mannheim), la RT-AMVbrl (Bethesda Research Laboratory), la RT-MMuLVss (Bethesda Research Laboratory), etc...

Comme indiqué ci-dessus, la transcriptase réverse doit être utilisée en quantité suffisante pour permettre, après traitement de dénaturation de l'ARN par la chaleur, l'obtention du premier brin d'ADNc dans les conditions choisies. Cette quantité suffisante correspond généralement à un excès par rapport à l'ADN polymérase.

La quantité (ou la concentration) de la transcriptase réverse peut être déterminée dans chaque cas par des expériences de routine. Elle peut varier par exemple 5 à 200 U, notamment 5 à 20 U pour RT-AMV et 100 à 200 U pour RT-MMuLV, pour un volume final de 100 µl. Généralement, le procédé de l'invention est caractérisé par le fait que l'on utilise des quantités de transcriptase réverse et d'ADN polymérase telles que leur rapport, exprimé en unités de RT-AMV et de Taq polymérase, respectivement, est au moins égal à 2, et en particulier au moins égal à 3, et est inférieur à 8, en particulier inférieur à 7, et peut varier notamment de 2 à 6, ou que, lorsqu'on choisit une transcriptase réverse et/ou une ADN polymérase autres que la RT-AMV et la Taq polymérase, respectivement, on utilise ces enzymes en quantités telles que des quantités équivalentes de RT-AMV et de Taq polymérase, respectivement, soient dans le même rapport au moins égal à 2 et inférieur à 8.

Après l'opération de transcription réverse, on peut dénaturer l'hétéroduplex ARN-ADNc formé par chauffage à une température suffisante, par exemple une température supérieure à 90°C. Bien entendu, il conviendra alors d'utiliser, en vue des opérations ultérieures d'amplification de l'ADN, un ADN polymérase capable de supporter une telle température, comme par exemple la Taq-polymérase, la rTth (Cetus), la Pfu DNA Polymérase (Stratagene), la Vent Polymérase (Biolabs), etc...

Après l'étape de dénaturation de l'hybride ARN-ADNc, on abaisse la température de façon à permettre l'hybridation de la seconde amorce (amorce 5'). Ici encore, cette température d'hybridation sera choisie de préférence assez élevée, par exemple au moins égale à 50°C, notamment de 50 à 80°C, de façon à permettre l'hybridation dans des conditions suffisamment discriminantes.

Les critères de choix de l'amorce 5' et de sa longueur sont évidemment les mêmes, avec les adaptations nécessaires, que ceux exposés ci-dessus pour le choix de l'amorce 3', et lorsque l'amorce 5' sera une amorce non strictement complémentaire de la cible, on pourra, comme précédemment, refroidir la solution à une température suffisamment basse (par exemple inférieure à 50°C, notamment entre 40 et 50°C) pour permettre l'hybridation de l'amorce 5' à une séquence d'ADN non rigoureusement complémentaire, pendant un temps suffisamment faible pour éviter des hybridations purement aléatoires ou non spécifiques, après quoi on réchauffera la solution jusqu'à une température prédéterminée choisie pour l'étape de synthèse du second brin d'ADNc.

Cette étape de synthèse du second brin d'ADNc s'effectue à une température suffisamment discriminante, c'est-à-dire une température suffisamment élevée mais restant compatible avec le maintien de l'hybridation spécifique de l'amorce 5', en particulier une température d'au moins 50°C (par exemple de 50 à 80°C environ). Les étapes d'hybridation de l'amorce sur le premier brin d'ADNc et de synthèse du second brin d'ADNc peuvent être effectuées à la même température.

Les amorces 3' et 5' peuvent être utilisées par exemple en quantités sensiblement équimolaires. Ces amorces, qui peuvent être constituées d'ARN ou d'ADN, sont utilisées en grand excès, comme cela est connu pour les réactions d'amplification. L'amorce 5' peut comporter à son extrémité 5' une queue oligonucléotidique non spécifique, comme on l'a déjà expliqué plus haut à propos de l'amorce 3'.

Après l'étape de synthèse du second brin d'ADNc, on peut soumettre l'ADNc double brin obtenu à un nombre suffisant de cycles d'amplification selon les méthodes connues, par exemple par dénaturation de l'ADN double brin, hybridation des amorces 3' et 5' sur les brins d'ADN néoformés dont elles sont respectivement complémentaires et synthèse de produit d'élongation sous l'action de l'ADN polymérase. De tels cycles d'amplification peuvent être effectués selon les méthodes connues par simples traitements thermiques à diverses températures, par exemple une température de 90-98°C environ pour la dénaturation de l'ADN double brin, une température de 40-80°C environ pour l'hybridation des amorces et une température de 60-80°C environ pour la synthèse des produits d'élongation. Ces cycles d'amplification, de même que l'ensemble des étapes du procédé de l'invention, peuvent être mis en oeuvre de façon automatique dans des appareils programmables du commerce.

Le procédé de l'invention est applicable notamment au diagnostic des infections virales ou bactériennes, et des désordres génétiques. Il est applicable aussi au typage bactérien ou viral, et au typage cellulaire, y compris le HLA. Il est applicable également au clonage d'endogènes non identifiés ou de séquences d'ARN inconnues théoriquement proches mais différentes de séquences connues en utilisant l'étape d'hybridation à température contrôlée, suffisamment basse, entre l'étape de dénaturation de l'ARN et la synthèse de l'ADNc. Le procédé de l'invention permet également d'amplifier de l'ARN *in situ* sur tissus (par exemple sur tissus fixés, sur tissus congelés ou sur frottis convenablement préparés) déposés ou fixés sur un support, le compartiment réactionnel étant par exemple délimité par la lame-support sur laquelle est fixé le tissu et une lame couvrante scellée, comme cela était réalisé pour l'amplification de l'ADN ; voir Nuovo G.J. et al., Am. J. Pathol., 139, 847-854 (1991).

Le procédé de l'invention peut être utilisé pour réaliser des co-amplifications, soit avec des systèmes d'amorces distincts, soit avec une amorce 3' commune.

Le procédé de l'invention peut notamment être utilisé pour réaliser la quantification d'une cible ARN, soit directement par rapport à une gamme étalon, soit par coamplification, selon les méthodes connues. La co-amplification est effectuée soit avec un standard interne introduit en quantité contrôlée et pouvant être amplifié à l'aide des mêmes amorces que l'ARN-cible : voir par exemple les documents PCT/US90/04707 et Michael Becker-André et al., Nucleic Acids Research, Vol.17, pp. 9437-9447 (1989), soit avec un ARN (gène marqueur) différent de l'ARN-cible et présent naturellement dans l'échantillon examiné ; voir notamment J. Chelly et al., Nature, Vol.333, pp. 858-860 (1988).

L'invention concerne également l'utilisation comme sonde (dans un procédé de détection du virus HIV1) ou comme amorce (dans un procédé d'amplification quelconque de l'ARN du virus HIV1, y compris un procédé tel que décrit dans la présente demande), d'une séquence oligonucléotidique:
- comprenant la séquence CCTATCTGTCCCCTCAGCTAC utilisable notamment comme amorce 3',
- ou comprenant la séquence TCTATCAAAGCAACCCAC utilisable notamment comme amorce 5'.

Les avantages procurés par l'utilisation de ces séquences ressortent de la partie expérimentale ci-après.

L'invention a également pour objet des oligonucléotides purifiés, comportant par exemple au plus 30 nucléotides environ, et contenant l'une des deux séquences qui viennent d'être définies. Lesdits oligonucléotides purifiés peuvent comporter, à au moins l'une des extrémités de la séquence minimum indiquée, des nucléotides qui peuvent être choisis parmi ceux entourant ladite séquence dans l'ADN complémentaire correspondant à l'ARN du VIH-1. Lesdits oligonucléotides purifiés peuvent également contenir à leur extrémité 5' une queue oligonucléotidique non spécifique, comme cela a été expliqué ci-dessus à propos des amorces d'amplification. Les oligonucléotides purifiés de l'invention peuvent également contenir des nucléotides modifiés et/ou marqués avec un agent traceur. Ils peuvent être utilisés notamment comme sondes de capture ou de détection dans une méthode de diagnostic des infections par le VIH-1, et/ou comme amorces d'amplification. Ils peuvent être préparés selon les procédés classiques de la synthèse d'oligonucléotides.

On va maintenant illustrer davantage l'invention à l'aide de la partie expérimentale donnée ci-après. Dans les exemples qui suivent, les amorces peuvent également être appelées "amplimers" ou encore "primers", et l'amorce 3' peut être également appelée "primer inverse". La technique de transfert d'ADN, dite transfert de Southern, depuis un gel, par exemple après électrophorèse, sur une membrane où l'ADN peut être hybridé avec une sonde nucléique marquée est désignée par l'expression "Southern Blot". Il en va de façon analogue pour la technique de transfert d'ARN dite transfert de Northern ; dans ces exemples, on fera référence aux dessins annexés dans lesquels:
- la fig.1 est un schéma de l'organisation des ARN messagers du VIH-1, avec indication des sondes ou amorces employées;
- la fig.2 représente le signal observé, en coups par minutes (cpm) après amplification selon la procédure décrite à l'exemple 1, en fonction du pourcentage initial de cellules infectées (deux manipulations indépendantes);
- la fig.3 est une image en autoradiographie obtenue après amplification et transfert de Southern selon le procédé de l'exemple 2 ; la colonne MPM (Marqueurs de poids moléculaire) représente des étalons de poids moléculaire; les nombres indiqués dans la colonne de gauche sont des nombres de nucléotides;
- les fig.4 et 5 représentent les images obtenues en autoradiographie respectivement après amplification et transfert de Southern respectivement dans les exemples 3 et 4 ;
- la fig.6 est un schéma des cycles de températures utilisés à l'exemple 5, avec indication du moment de l'introduction des réactifs ;
- la fig.7 est l'image obtenue en autoradiographie après amplification et transfert selon le procédé de l'exemple 5 ; les références portées sur la fig.7 correspondent aux divers modes opératoires indiqués à la fig.6;
- la fig.8 est l'image en autoradiographie obtenue à l'issue du procédé de l'exemple 6 ;
- la fig.9 est l'image en autoradiographie obtenue à l'issue du procédé de l'exemple 7; les références des diverses colonnes correspondent aux quantités (nM) d'amorce 3';
- la fig.10 représente l'image en autoradiographie obtenue à l'issue du procédé de l'exemple 8 ; les références des colonnes correspondent aux quantités d'ARN de l'échantillon analysé (en ng) ; le trait correspondant à la bande observée sur la photographie originale de la colonne 2.5 a été renforcé pour être davantage visible sur les reproductions de la fig. 10;
- la fig. 11 représente les images d'autoradiographie obtenues à l'issue des diverses expériences décrites à l'exemple 9 (chapitre "Identification de fragments amplifiés");
- la fig.12 représente les images obtenues en autoradiographie dans les expériences décrites à l'exemple 9 (chapitre "Qualité de l'amplification") ; et
- les fig.13 et 14 représentent les images obtenues en autoradiographie dans les expériences décrites aux exemples 10 et 11, respectivement.

### PARTIE EXPERIMENTALE

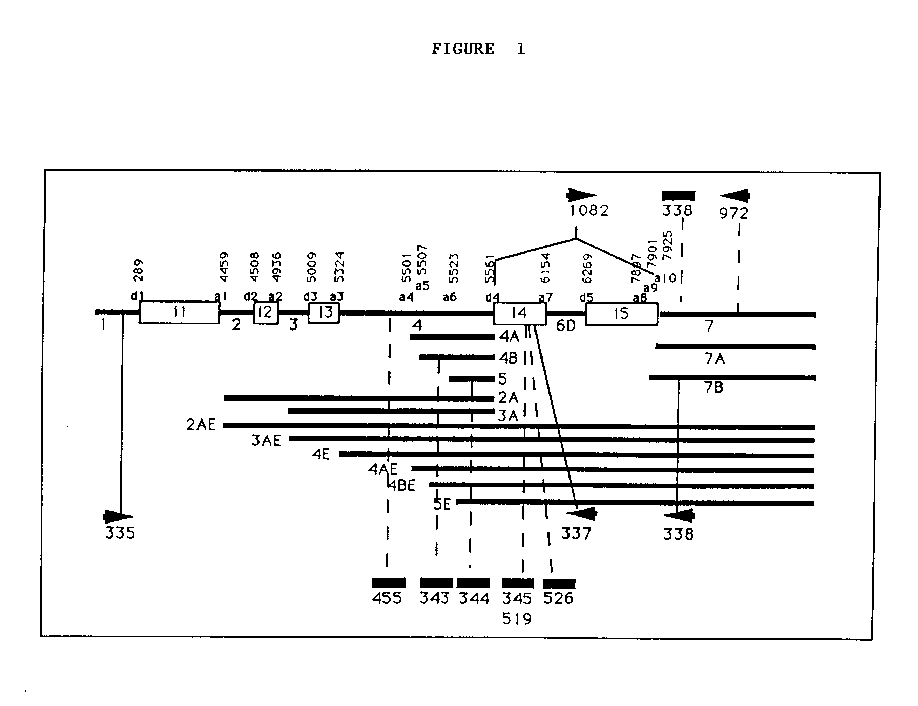

Le modèle biologique étudié dans cette partie expérimentale est l'ARN du virus de l'immunodéficience humaine, VIH-1. La structure des ARNm du virus et en particulier la localisation des sites donneurs (d1 à d4) et accepteurs (a1 à a10) d'épissages, la nomenclature des exons (traits gras marqués 1à 7 avec caractérisation A,B,D,AE,BE ou E) et des introns (i4 à i5) ainsi que la localisation des amplimers 5' (flèche de gauche à droite), des primers inverses/amplimers 3' (flèche de droite à gauche) et des sondes (blocs gras) sont répertoriés dans la figure1. Les amorces et sondes oligonucléotidiques ont été synthétisées sur un synthétiseur Applied Biosystem 394 DNA/RNA, analysées et purifiées en HPLC phase inverse lorsque cela était requis. Elles sont répertoriées dans le tableau 1 ci-dessous. La numérotation utilisée dans la figure 1 et le tableau suit la nomenclature du clone moléculaire HIV-HXB2R (Lawrence, J., et al. 1990., edited by Myers, G., et al. ).

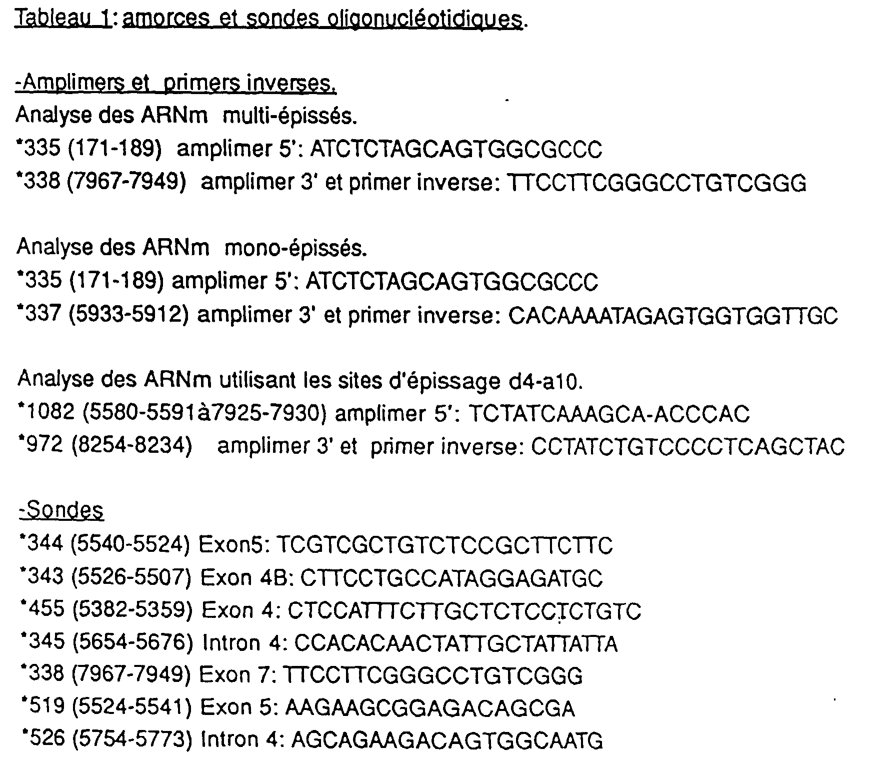

### EXEMPLE 1: Efficience et Sensibilité de la méthode en une étape.

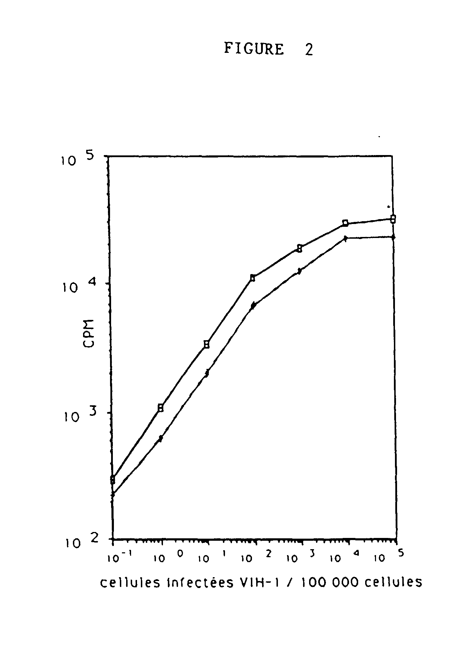

Pour déterminer la sensibilité de la méthode d'amplification en une étape unique de manipulation, nous avons défini un couple d'amplimers, à l'aide des jonctions des sites accepteurs d4-a8 du virus VIH-1 (Tat exons1-4-7, Rev exons 1-4A-7, Nef exons 1-5-7, figure 1), conduisant à l'amplification spécifique d'ARNms multi-épissés du virus VIH-1 (l'aspect épissage du VIH-1 sera développé ultérieurement dans l'exemple 9) . 1µl de ARN guard (Pharmacia LKB Biotechnology) est ajouté, comme inhibiteur de RNAse, à tous les échantillons, ceux-ci contenant 1 µg d'ARN. Les ARN ont été extraits à partir de la lignée lymphoïde H9 infectée par la souche HTLV-IIIB, par la technique dite du guanidium isothiocyanate (Chirgwin J.M., et al. 1979. Biochem 18:5294-5299.) suivie d'une ultracentrifugation sur gradient de chlorure de césium (Gilsin V., et al. 1973. Biochem 13:2633-2638.). La concentration finale du tampon utilisé est 10mM Tris pH 8.3, 50 mM KCl, 1.5 mM MgCl2, 0.01% gélatine. Les concentrations finales sont: amplimers 310 nM, dNTPs 250 µM,Taq polymérase (Perkin Elmer-Cétus) 2.5U, RT-AMV (Boehringer-Mannheim) 10U. Le protocole d'amplification, réalisé dans un thermo-cycleur, consiste en une incubation de 10 minutes à 65°C (dénaturation de l'ARN et hybridation du primer inverse lui-même identique à l'amplimer 3'), puis 8 minutes à 50°C (réverse transcription), puis 5 minutes à 95°C (dénaturation des hybrides ADN/ARN et inactivation de la réverse transcriptase), puis consécutivement, 35 cycles de PCR sont alors réalisés, un cycle consistant en 1 minute à 95°C pour la dénaturation de l'ADN, 1 minute à 55°C pour l'hybridation des amplimers sur les matrices ADN monocaténaires, et 1 minute à 72°C pour l'élongation des amorces oligonucléotidiques (amplimers); le dernier cycle présente de plus une extension de 7 minutes de l'étape d'élongation afin de terminer la synthèse des fragments d'ADN incomplets. Les produits de RT-PCR sont ensuite analysés en southern blot. Ils sont analysés sur gel d'acrylamide 5% puis l'ADN est dénaturé à la chaleur par immersion du gel dans de l'eau bouillante pendant 5 minutes, puis le gel est équilibré dans un tampon de transfert (Tris base 89 mM, acide borique 89 mM, EDTA 2 mM, pH 8,0). L'ADN subit alors un transfert électrique (1 heure, 300 mA, 4°C) sur membrane de nylon et est fixé aux UV. La préhybridation est réalisée pendant une nuit à 42°C dans un tampon 5xSSC, 0.1% SDS, Denhart 5X, ADN de sperme de saumon 0.2 mg/ml, formamide désionisée 18%, et l'hybridation est réalisée pendant 4 heures à 50°C, dans le susdit tampon, contenant 10⁶ cpm/ml de la sonde oligonucléotide marquée au ddATP 32P à l'aide de l'enzyme terminale transférase (Boehringer Mannheim), à une activité spécifique de 10⁸ cpm/mg. Les filtres sont alors lavés en 1xSSC, 0.1 % SDS, 10 minutes à température ambiante et 10 minutes à 50°C, puis développés par autoradiographie 3 heures à -80°C. L'analyse en southern blot d'ARNs amplifiés par les primers 1082-972 (voir tableau1 et figure 1) met en évidence en autoradiographie la bande de 340 pb attendue, révélée avec la sonde 338 (tableau 1, figure 1). Aucun signal n'a pu être observé, que ce soit en absence de RT ou lorsque la PCR a été réalisée en présence de 1 µg d'ADN, extrait de cellules infectées HTLV-IIIB, comme cela était attendu. Une bande faible, observée après 4 heures d'autoradiographie à -80° C, prouve que l'ARN spécifique du VIH peut être détecté quand une cellule sur 10⁶ est infectée, en utilisant la méthode en une étape selon l'invention. Les rendements d'extraction permettent d'affirmer que l'ARN d'environ 0.1 cellules H9 infectée est présent dans l'échantillon (1 µg d'ARN) résultant de la dilution finale utilisée pour la RT-PCR. Le seuil de sensibilité de l'essai est donc vraisemblablement compris entre 10 et 100 copies d'une cible donnée. L'essai en une étape a été aussi testé pour la quantification potentielle d'ARN, à l'aide d'une dilution séquentielle d'un facteur dix des cellules infectées. Une courbe de PCR typique, représentant la quantité de signal généré en fonction de la proportion de cellules infectées a été déterminée (figure 2): le résultat indique que, dans ces conditions expérimentales, le signal obtenu est corrélé à la concentration initiale d'ARNm cible.

### EXEMPLE 2: Comparaisons de plusieurs RT-AMV avec plusieurs RT-MMuLV.

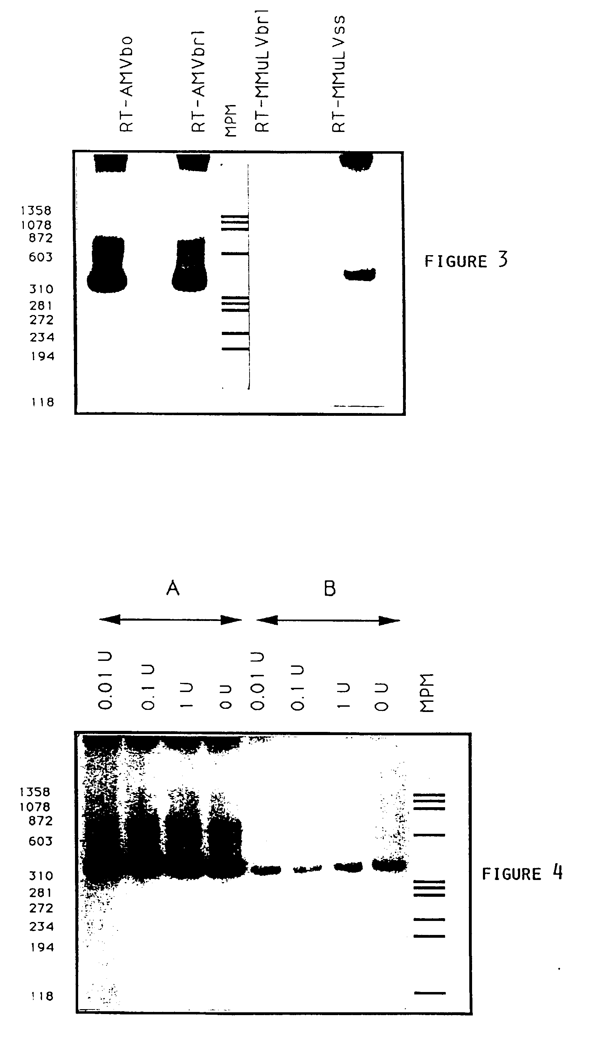

Les conditions expérimentales utilisées sont essentiellement identiques à celles décrites dans l'exemple 1. L'ARN est extrait de cellules H9 infectées par la souche HTLV-IIIB, les primers utilisés sont 335 et 337, les enzymes sont la RT-AMVbm (Boehringer-Mannheim), à 10 U par réaction, la RT-AMVbrl (Bethesda Research Laboratory), à 10 U par réaction, la RT-MMuLVbrl (Bethesda Research Laboratory), à 200 U par réaction, la RT-MMuLVss clonée et ne possédant pas d'activité RNAseH (Bethesda Research Laboratory), à 200 U par réaction, les cycles de température étant de 10 minutes à 65°C, 8 minutes à 50°C, 5 minutes à 95°C, puis consécutivement, 35 cycles de PCR sont alors réalisés, un cycle consistant en 1 minute à 95°C, 2 minutes à 55°C, et 2 minutes 30 secondes à 72°C; le dernier cycle présente de plus une extension de 7 minutes de l'étape d'élongation. L'analyse en southern blot (cf exemple 1) d'ARNs amplifiés par les primers 335-337 (voir tableau1 et figure 1) met en évidence la bande majoritaire attendue à 530 pb (figure 3), révélée avec la sonde 526 (tableau 1, figure 1). Les intensités respectives des signaux montrent la plus grande efficacité de la RT-AMV, quelle que soit sa source d'obtention.

### EXEMPLE 3: Influence de l'ajout de RNAseH avec la RT-AMV.

Les conditions expérimentales utilisées sont essentiellement identiques à celles décrites dans l'exemple 1. L'ARN est extrait de cellules H9 infectées par la souche HTLV-IIIB, les cellules infectées représentant soit 100% (A) soit 1% (B) de la population traitée. Les primers utilisés sont 335-337, la quantité de RNAseH (Boehringer Mannheim) introduite est de 1 Unité, 0.1 Unité, 0.01 Unité et 0 Unité comme contrôle. Les cycles de température utilisés sont identiques à ceux décrits dans l'exemple 2. L'analyse en southern blot (cf exemple 1) d'ARNs amplifiés par les primers 335-337 (tableau1, figure 1) met en évidence la bande attendue à 530 pb (figure 4), révélée avec la sonde 526 (tableau 1, figure 1). Aucune différence notable n'est observée, indépendamment de la quantité initiale d'ARN cible. Remarquons également que, indépendamment du fait que la RT-MMuLVss (RNAseH moins) soit moins efficace que les RT-AMV (exemple 2 figure 3), elle est cependant notablement plus efficace que les RT-MMuLV non clonées présentant une activité RNAseH (exemple 2 figure 3). L'ensemble de ces résultats montre que l'efficacité de la méthode n'est pas due à une activité RNAseH présente dans le tube réactionnel.

### EXEMPLE 4: Cinétique de température.

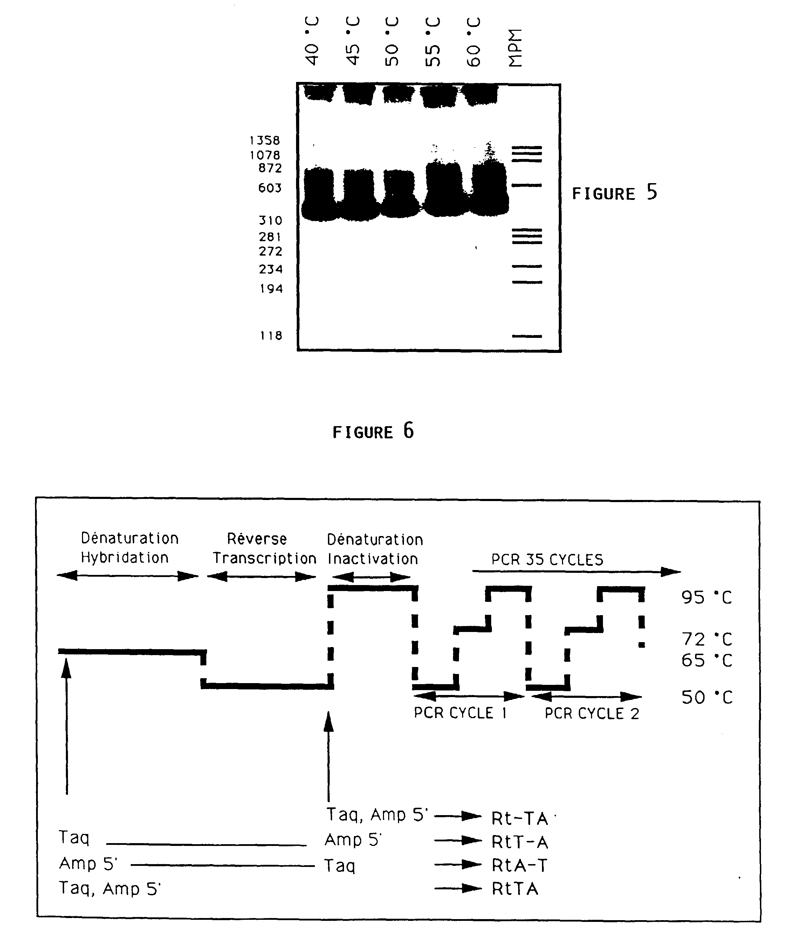

Les conditions expérimentales utilisées sont essentiellement identiques à celles décrites dans l'exemple 1. L'ARN est extrait de cellules H9 infectées par la souche HTLV-IIIB, les primers utilisés sont 335-337. Le cycle d'amplification, réalisé dans un thermo-cycleur consiste en une incubation de 10 minutes à 65°C, puis la réverse transcription est réalisée à 8 minutes à différentes températures, respectivement 40°C, 45°C, 50°C, 55°C, 60°C, puis 5 minutes à 95°C, et consécutivement, 35 cycles de PCR sont alors réalisés, un cycle consistant en 1 minute à 95°C, 2 minutes à 55°C, et 2 minutes 30 secondes à 72°C; le dernier cycle présente de plus une extension de 7 minutes de l'étape d'élongation. L'analyse en southern blot (cf exemple 1) d'ARNs amplifiés par les primers 335-337 (tableau1, figure 1) met en évidence la bande attendue à 530 pb (figure 5), révélée avec la sonde 526 (tableau 1, figure 1). Les intensités respectives des signaux ne montrent pas une décroissance notable de l'efficacité de l'amplification, et donc de l'efficacité de la RT-AMV, dans une gamme de température comprise entre 40 °C et 60°C, pendant 8 minutes, en plus du cycle de dénaturation à 65°C pendant 10 minutes.

### EXEMPLE 5: Positionnement de la Taq et de l'amplimer 5' dans la réaction d'amplification.

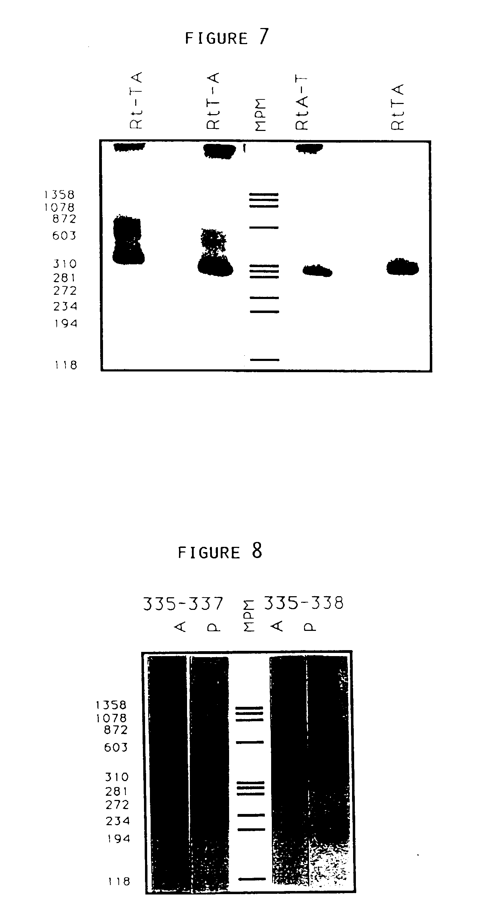

Les conditions expérimentales utilisées sont identiques à celles décrites dans l'exemple 1. L'ARN est extrait de cellules H9 infectées par la souche HTLV-IIIB, les primers sont 335-337, la Taq et/ou l'amplimer 5' étant introduits dans le tube réactionnel soit dès le début de la réaction, soit à la fin de l'étape de synthèse de l'ADNc de 8 minutes à 50°C. Les quatre combinaisons ont été réalisées et sont explicitées sur le schéma de la figure 6. Les cycles de température utilisés sont identiques à ceux décrits dans l'exemple 2. L'analyse en southern blot (cf exemple 1) d'ARNs amplifiés par les primers 335-337 (tableaul, figure 1) met en évidence la bande majoritaire attendue à 530 pb (figure 7), révélée avec la sonde 526 ( tableau 1, figure 1). Aucune différence significative est observée, quels que soient le moment d'introduction de la Taq et / ou de l'amplimer 5'.

### EXEMPLE 6: influence du moment d'introduction de la RT

Les conditions expérimentales utilisées sont essentiellement identiques à celles décrites dans l'exemple 1. L'ARN est extrait de cellules H9 infectées par la souche HTLV-IIIB, les primers sont 335-337 ou 335-338. Les cycles de température utilisés sont identiques à ceux décrits dans l'exemple 2. La réverse transcriptase est présente dans le tube réactionnel dès le début de la réaction (A) ou introduite après l'étape de dénaturation/hybridation à 65°C (P). L'analyse en southern blot (cf exemple 1) d'ARNs amplifiés par les primers 335-337 ou 335-338 (tableaul, figure 1) met en évidence les bandes caractéristiques attendues (figure 8), révélées avec la sonde 519 (tableau 1, figure 1). Dans le cas où la RT est introduite après l'étape de dénaturation, on peut remarquer en particulier pour l'amplification d'ARNm fortement structurés (335-338, colonne P) un accroissement des signaux aspécifiques dont l'interprétation sera développée ultérieurement (cf exemple 9).

### EXEMPLE 7: influence de la quantité de primer 3' (primer inverse et amplimer)

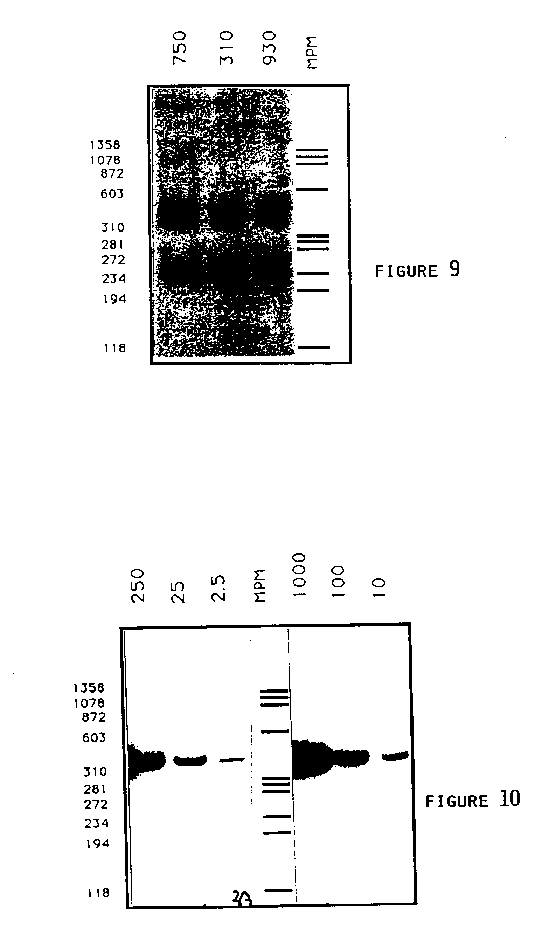

Les conditions expérimentales utilisées sont essentiellement identiques à celles décrites dans l'exemple 1. L'ARN est extrait de cellules H9 infectées par la souche HTLV-IIIB, 250 ng d'ARN sont utilisés comme substrat réactionnel, les primers sont 335-338, la quantité de primer inverse (338) étant soit de 310 nM (équimolaire avec l'amplimer 5'), soit de 930 nM. Un essai a également été réalisé en utilisant l'enzyme rTth avec une concentration de750 nM du primer inverse et de 150 nM de l'amplimer 5', selon une méthodologie décrite dans le paragraphe Il ( Myers T.W., et al. 1991. Biochem 30:7661-7666. ). Les cycles de température utilisés sont identiques à ceux décrits dans l'exemple 2. L'analyse en southern blot (cf exemple 1) d'ARNs amplifiés par les primers 335-338 (tableau1, figurel) met en évidence les bandes caractéristiques attendues (figure 9), révélée avec la sonde 519 (tableau 1, figure 1). Aucune différence tant qualitativement que quantitativement n'est observée, quelle que soit la quantité de primer inverse. Il ne semble donc pas nécessaire d'utiliser celui-ci en excès par rapport à l'amplimer 5'.

### EXEMPLE 8: influence de la quantité d'ARN dans le tube réactionnel.

Les conditions expérimentales utilisées sont identiques à celles décrites dans l'exemple 1. L'ARN est extrait de cellules H9 infectées par la souche HTLV-IIIB, les cellules infectées représentant 0.01% de la population traitée. La quantité d'ARN total utilisée est de 2.5, 10, 25, 100, 250 et 1000 ng. L'analyse en southern blot (cf exemple 1) d'ARNs amplifiés par les primers 1082-972 (tableau1, figure 1) met en évidence la bande 340 pb attendue (figure 10), révélée avec la sonde 338 (tableau 1, figure 1). Le signal obtenu est proportionnel à la quantité initiale d'ARN cible et semble indépendant de la quantité totale d'ARN présente dans le tube réactionnel.

### EXEMPLE 9: comparaison des paramètres réactionnels impliqués dans la RT-PCR.

On a comparé les efficacités relatives des méthodes dites en "une étape" ou en "deux étapes", en utilisant à la fois la RT et la Taq.

Dans la méthode en "deux étapes", nous avons étudié l'influence des paramètres volume, tampon et température sur l'hybridation du primer inverse ainsi que sur la transcription inverse. Les différents protocoles de RT-PCR comprenant en outre l'ensemble des constituants impliqués dans la réaction, leur concentration ainsi que l'ordre séquentiel de leur introduction sont répertoriés dans le tableau 2. 1µl de ARN guard (Pharmacia LKB Biotechnology) est ajouté à tous les échantillons, ceux-ci contenant 1 µg d'ARN. Les ARN ont été extraits à partir de la lignée promonocytique U937 infectée par la souche VIH-LAV.

Dans le tableau 2, chaque colonne correspond à un mode de réalisation particulier utilisé, selon les amorces employées, soit pour l'amplification de la région Reg, soit pour l'amplification de la région Env. Chaque numéro d' échantillon, arbitraire, correspond à une expérience particulière. Dans tous les cas, la dénaturation de l'ARN est effectuée par chauffage pendant 10 minutes à 65°C. Elle est suivie soit d'un refroidissement lent (+) en 30 minutes à 42°C, soit la réaction est immédiatement enchaînée avec l'étape de réverse transcription (e) ou le tube est refroidi rapidement dans la glace (-). Pour la réaction de synthèse du premier brin d'ADNc (Extension), on a indiqué sur la première ligne le temps en minutes (sauf indications contraires) et sur la seconde ligne la température en °C.

**Tableau 2:**

| Description des procédures de RT-PCR. | | | | | | | | | |
|---|---|---|---|---|---|---|---|---|---|
| **Echantillon Reg** | 87 | 88 | K8 | 81 | 82 | 83 | 84 | 85 | 86 |
| **Env** | 77 | 78 | K7 | 71 | 72 | 73 | 74 | 75 | 76 |

| **HYBRIDATION (ETAPE 1)** | | | | | | | | | |
|---|---|---|---|---|---|---|---|---|---|
| ARN (µg) | 1 | 1 | 1 | 1 | 1 | 1 | 1 | 1 | 1 |
| TAMPON | eau | eau | PCRx1 | PCRx1 | PCRx1 | PCRx1 | PCRx1 | PCRx1 | PCRx1 |
| MgCl2 (mM) | 0 | 0 | 1.5 | 1.5 | 1.5 | 1.5 | 1.5 | 1.5 | 1.5 |
| dNTP(µM) | 0 | 0 | 250 | 250 | 250 | 250 | 250 | 250 | 250 |
| amplimer 3'(nM) | 1550 | 1550 | 310 | 310 | 310 | 310 | 310 | 310 | 310 |
| amplimer 5' (nM) | 0 | 0 | 310 | 0 | 0 | 0 | 0 | 0 | 0 |
| Taq:RT-AMV(U) | 0 | 0 | 2.5:10 | 0 | 0 | 0 | 0 | 0 | 0 |
| Volume Final (µl) | 20 | 20 | 100 | 97 | 97 | 97 | 97 | 97 | 97 |
| **Dénaturation** | .............................10 min.-65°C....................................... | | | | | | | | |
| **Refroidissement** | - | - | e | + | + | + | - | - | - |

| **REVERSE TRANSCRIPTION (ETAPE 2)** | | | | | | | | | |
|---|---|---|---|---|---|---|---|---|---|
| TAMPON | PCRx1 | PCRx3 | PCRx1 | PCRx1 | PCRx1 | PCRx1 | PCRx1 | PCRx1 | PCRx1 |
| MgCl₂ (mM) | 1.5 | 4.5 | 1.5 | 1.5 | 1.5 | 1.5 | 1.5 | 1.5 | 1.5 |
| dNTP (µM) | 250 | 750 | 250 | 250 | 250 | 250 | 250 | 250 | 250 |
| AMV (10U) | + | + | ajouté étape1 | + | + | + | + | + | + |
| Volume Final (µl) | 98 | 33 | 100 | 98 | 98 | 98 | 98 | 98 | 98 |
| **Extension** | 1h | 1h | 8 | 10 | 1h | 45* | 10 | 1h | 45* |
| | 45 | 45 | 50 | 50 | 45 | 42 | 50 | 45 | 42 |
| **Dénaturation** | ..............................5 min.-95°C....................................... | | | | | | | | |

| **PCR (ETAPE 3)** | | | | | | | | | |
|---|---|---|---|---|---|---|---|---|---|
| TAMPON | PCRx1 | PCRx1 | PCRx1 | PCRx1 | PCRx1 | PCRx1 | PCRx1 | PCRx1 | PCRx1 |
| MgCl₂ (mM) | 1.5 | 1.5 | 1.5 | 1.5 | 1.5 | 1.5 | 1.5 | 1.5 | 1.5 |
| dNTP(µM) | 250 | 250 | 250 | 250 | 250 | 250 | 250 | 250 | 250 |
| Taq 2.5U | + | + | ajouté étape 1 | + | + | + | + | + | + |
| amplimer5' (310nM) | + | + | ajouté étape1 | + | + | + | + | + | + |
| Volume Final (µl) | 100 | 100 | 100 | 100 | 100 | 100 | 100 | 100 | 100 |

| | | | | | | | | | |
|---|---|---|---|---|---|---|---|---|---|
| * suivi de 15 minutes à 50°C | | | | | | | | | |

Chaque échantillon est stocké dans la glace avant tout apport de réactifs lorsque ceci est requis. L'hybridation du primer inverse (étape 1) est réalisée dans de l'eau ou dans un tampon 10mM Tris pH 8.3, 50 mM KCl, 1.5 mM MgCl2, 0.01% gélatine (appelé PCRX1) ou dans un tampon 30 mM Tris pH 8.3, 150 mM KCl, 4.5 mM MgCI2, 0.01% gélatine (appelé PCRx3), à 65°C, avec ou sans refroidissement lent à 42°C. Les primers inverses, 338 (tableau 1, figure 1) pour les ARN multi-épissés (appelés Reg dans le tableau 2), 337 (tableau 1, figure 1) pour les ARN mono-épissés (appelés Env dans le tableau 2), sont choisis comme étant identiques à l'amplimer 3' impliqué dans la réaction de PCR. L'étape de réverse transcription est réalisée en présence de concentrations de MgCI2 et de dNTPs adaptées soit à l'activité de la réverse transcriptase soit de la Taq polymérase, en faisant varier à la fois le temps (de 8 minutes à 1 heure) et la température (42°C à 50°C) de la réaction de synthèse de l'ADNc. La concentration de la RT-AMV (Boehringer Mannheim) est de 10U pour 1 µg d'ARN. Avant l'enchainement des cycles de PCR, les hybrides ADNc/ARNm sont dénaturés et la réverse transcriptase inactivée par chauffage de l'échantillon à 95°C pendant 5 minutes. 35 cycles de PCR sont alors réalisés, un cycle consistant en 1 minute à 95°C pour la dénaturation de l'ADN, 2 minutes à 55°C pour l'hybridation des amplimers sur les matrices ADN monocaténaires, et 2 minutes à 72°C pour l'élongation des amorces oligonucléotidiques; le dernier cycle présente de plus une extension de 7 minutes de l'étape d'élongation afin de terminer la synthèse des fragments d'ADN incomplets. La concentration de la Taq polymérase (Perkin Elmer-Cetus) est de 2.5U pour 1µg d'ARN initial. Dans tous les cas, la réaction de PCR est réalisée dans le tampon 10mM Tris pH 8.3, 50 mM KCl, 1.5 mM MgCl2, 0.01% gélatine.

Nous avons choisi l'ARNm du VIH-1 comme modèle parce qu'il est connu pour présenter une stratégie complexe de l'épissage ( Schwartz S., et al. 1990a. J Virol 64 (6):2519-2529.) liée au type cellulaire (Robert-Guroff M., et al. 1990. J Virol 64 (7):3391-3398. ) et à l'état de différenciation de la cellule. L'épissage différentiel conduit à trois classes d'ARNm, non-épissés, mono-épissés et multi-épissés, qui peuvent être identifiées respectivement en Northern blot par trois bandes majeures respectivement de 9.5, 4.3 et 1.8-2 Kb. Cependant, les ARNm du VIH forment une famille complexe comprenant plus de 20 ARNm, et seuls des outils très performants tels que la méthode de protection par des ribonucléases ou la RT-PCR peuvent permettre de discriminer entre plusieurs ARNm dans une même classe. Une difficulté supplémentaire concerne les structures secondaires de fortes stabilités situées dans les ARNm rétroviraux et qui pourraient compromettre la réaction de synthèse de l'ADNc.

### 1/ Identification de fragments amplifiés

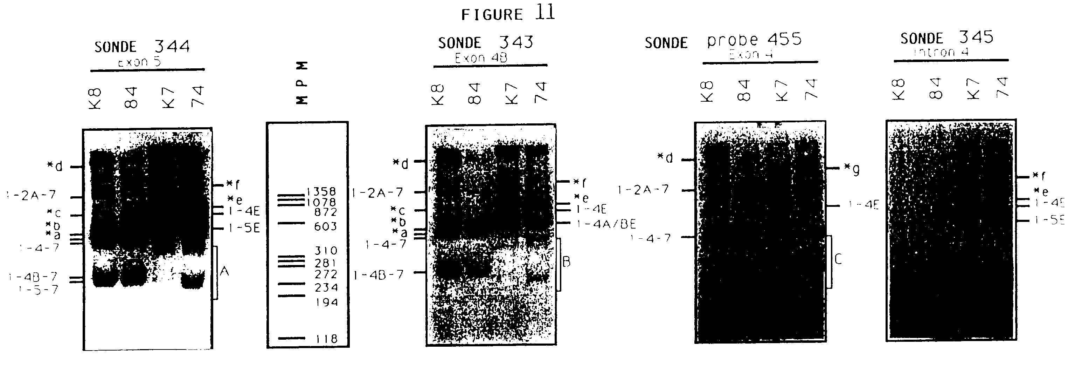

Le typage phénotypique des ARNm de VIH a été réalisé à l'aide de primers spécifiques des classes décrites précédemment, choisis dans des exons séparés, respectivement 337-335 (tableau 1, figure 1) pour l'identification de ARNm mono-épissés et 338-335 (tableau1, figure 1) pour la caractérisation de ARNm multi-épissés. Cette localisation de primers ne permet pas l'amplification d'ADN potentiellement contaminant et d'ARN génomique. La même analyse a été réalisée à la fois pour les essais en une étape (tableau 2, colonnes K8 et K7) et en deux étapes (tableau 2, colonnes 84 et 74). Les amplimers, primer inverses et sondes oligonucléotidiques spécifiques des divers introns et exons sont répertoriés dans le tableau 1 et localisés sur la figure 1. Sur la figure 11 est indiquée en face de chaque bande son équivalence en jonction d'exons, la nomenclature utilisée étant celle décrite sur la figure 1. L'analyse en southern blot (cf exemple 1) d'ARNm multi-épissés amplifiés à l'aide des amorces 335-338 a mis en évidence un modèle complexe qui peut être résolu grâce à des sondes discriminantes (figure 11, colonnes K8 et 84). Les ARNm mono-épissés n'ont pas été amplifiés, comme cela était attendu, par les primers 335-338, (figure 11, sonde 345, colonnes K8 et 84) car le fragment d'acide nucléique est trop long pour être amplifié dans ces conditions de PCR. L'ARN amplifié par les primers 335-338 (figure 11, colonnes K8 et 84) a produit un modèle complexe comprenant 4 bandes qui pourraient être interprétées comme des ARNm codant pour les protéines Nef, Rev, Tat et Vif ( Schwartz S., et al. 1990a. J Virol 64 (6):2519-2529.; Guatelli J.C., et al. 1990. J Virol 64 (9):4093-4098. ; Robert-Guroff M., et al. 1990. J Virol 64 (7):3391-3398. ). La bande inférieure d' environ 231 pb, correspondant aux exons 1-5-7, codant pour la protéine Nef, a été révélée par la sonde 344 (exon 5) mais pas par les sondes en amont (343, 455) ni par la sonde intron Env (345). La bande immédiatement supérieure, d' environ 247 pb, révélée à la fois par les sondes 344 (exon 5) et la sonde 343 (exon 4B), mais négative avec les sondes 455 (exon 4) et 345 (intron 4), correspond à l'ARNm codant pour Rev suivant les jonctions exon 1-4B-7. Les bandes supérieures, d'environ 430 à 1295 pb, révélées par toutes les sondes, sauf celle correspondant à l'intron env (345) peuvent correspondre aux exons 1-4-7 et 1-2A-7, codant respectivement pour les ARNms Tat et Vif. Les bandes supplémentaires (*a, *b, *c, *d) sont difficiles à interpréter. Les bandes appelées *a et *b peuvent être le résultat de l'utilisation des deux sites accepteurs a4 et a5 (révélés par les sondes 344 et 343, mais négatifs avec la sonde 455) et peuvent représenter les ARNms 1-3-4A-6D-7 (422 pb) et 1-3-4B-6D-7 (436 pb) pouvant coder pour un ARNm codant pour une protéine présentant une activité de type Rev, comme dans le cas de la protéine Tev (Benko D.M., et al. 1990. J Virol 64:2505-2518.). La bande ∗c d'environ 730 pb est interprétée comme un ARNm utilisant les sites accepteurs de Rev (a4 et a5).

L'analyse en southern blot (cf exemple 1) d'ARN amplifiés par les primers 335-337 (figure 11, colonnes K7 et 74), a mis en évidence deux bandes pouvant être interprétées comme des ARNms codant pour les protéines Env/Vpu et Tat (Schwartz S., et al. 1990b. J Virol 64 (11):5448-5456.). La bande inférieure d'environ 530 pb pourrait être l'ARNm de EnvNpu, correspondant aux exons 1-5E, révélés à la fois par les sondes 345 (exon 4) et 344 (exon 5), mais négatifs pour les sondes présentes en amont (343 et 455). La bande immédiatement supérieure, d'environ 729 pb, révélée par les sondes 345 (intron 4), 455 (exon 4), 343 (exon 4B) et 344 (exon 5), peut être interprétée comme un ARNm 1-4E codant pour la protéine Tat. Trois bandes appelées *e, *f et *g n'ont toujours pas été expliquées. La réactivité avec les sondes 343 et la non-réactivité avec la sonde 455 sembleraient prouver que les ARNms *e et *f utilisent les sites accepteurs de Rev. De plus, de nombreuses bandes inattendues ont été observées dans la colonne 74, à l'aide des sondes 344, 343, 455 (parenthèses A, B, C). La distribution typique de ces bandes, suivant le signal des sondes et leur répartition, a permis de les identifier comme résultant de l'amplification des ARNms multi-épissés (cf paragraphe précédent, amplification 335-338). Ces bandes peuvent être identifiées par l'absence de signal avec la sonde intron d'Env (345) et par la perte des bandes typiques ressemblant à celles de Nef et Rev, utilisant respectivement les sondes 343 et 455. En outre, du fait que leur taille est d'environ 20 pb inférieure à celle obtenue lors de l'amplification des ARNm multi-épissés originaux, ces bandes ne résultent pas de la contamination par le primer 338. Juste en amont de la région du primer 338 se trouve la séquence suivante AAA / ACCCACCTCCCAACCCCGAG (exon 5 / exon 7) qui présente des similitudes (soulignées) avec le primer Env inverse 337 GCAACCACCACTCTATTTTGTG, incluant 11 puissantes interactions potentielles (A-T, G-C) avec un noyau central riche en C et une interaction faible (G-T) à la terminaison 3'. Nous pouvons alors nous attendre à ce que, sous des conditions non-optimales de la transcription inverse, un ADNc soit généré, et à ce que dans la PCR subséquente, ce signal non spécifique soit considérablement amplifié. Nous avons d'ailleurs observé ce phénomène lors de l'étude de différentes conditions de RT-PCR décrites ci-dessous.

### 2/ Qualité de l'amplification

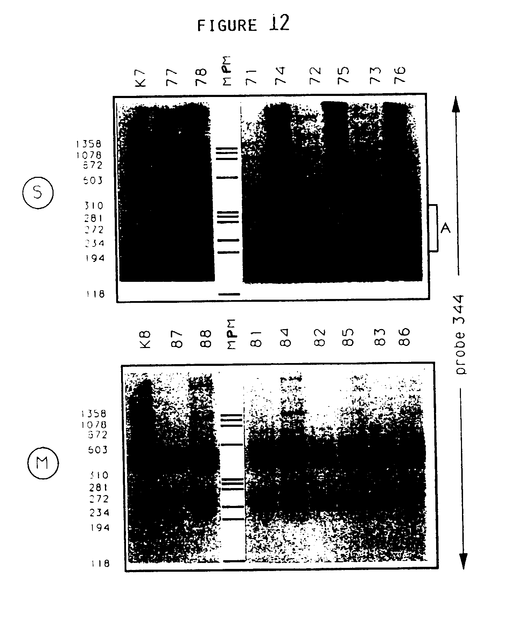

La RT-PCR inclut trois étapes, successivement l'hybridation du primer inverse avec ARN, la synthèse ADNc et l'amplification PCR. De nombreux protocoles décrivent différentes conditions pour chacune de ces trois étapes, notamment les étapes d'hybridation et de transcription inverse. Nous avons donc testé différentes façons de réaliser la RT-PCR (tableau 2), comprenant différentes conditions pour l'hybridation du primer inverse (Hybridation, étape 1) et la synthèse de l'ADNc (Reverse transcription, étape 2), suivies d'une PCR standard (PCR étape 3). Les données répertoriées sur le tableau 2 concernent la composition du tampon réactionnel, le volume d'incubation, le temps et la température de réaction, les concentrations de dNTP, les concentrations et le moment d'introduction des amplimers, de la Taq polymérase et de la réverse transcriptase. Les ARNm multi-épissés (tableau 2: Reg, figure 12: M) et les ARNm mono-épissés (tableau 2: Env, figure 12 S), amplifiés respectivement avec les primers 335-338 et 335-337, ont été analysés par southem blot avec la sonde 344 (figure 12).

### 2-1/ Hybridation

### Effet de la température:

Durant nos expériences, l'hybridation du primer inverse a montré qu'après 10 minutes de dénaturation d'ARN à 65° C, le refroidissement lent à 42° C avait un effet néfaste sur le résultat attendu, aussi bien qualitativement que quantitativement. La comparaison (figure 12S) des colonnes 71/74, 72/75 et 73/76, pour l'amplification des ARNm mono-épissés, a mis en évidence une augmentation de signaux inattendus (parenthèse A), de poids moléculaire faible (voir ci-dessus : identification de fragments amplifiés) correspondant à une baisse du signal spécifique majeur (EnvNpu ARNm 1-5E à 530 pb). Une telle baisse du signal spécifique peut aussi être observée après amplification d'ARNm multi-épissés (figure 12M 81/84, 82185, 83/86), bien que moins importante et sans apparition de bandes aspécifiques. Ceci suggère que le refroidissement lent favorise la formation de structures secondaires stables, interférant avec l'étape de RT. Ceci est plus frappant dans le cas des ARNm mono-épissés, du fait de la grande stabilité de leur structure secondaire, dans la région de fixation de la protéine Rev (Rev Responsive Element: RRE 7325-7531) (Holland S.M., et al. 1990. J Virol 64 (12):5966-5975.) située dans l'intron Env et décrite comme une structure secondaire très stable.

### 2-2/ Transcription inverse

### Influences de la température d'hybridation, et des concentration dNTPs et MgCI2

L'efficacité de la RT-PCR est améliorée avec l'augmentation de la température pour la réaction de RT, comme indiqué par l'augmentation du signal spécifique (figure 12S : 74 > 75 > 76 et figure 12M : 84 > 85 > 86). Une synthèse d'ADNc de 45 minutes, suivie d'une élongation à 50° C, a occasionné des perturbations qualitatives et quantitatives, en comparaison avec le chauffage direct à 50° C. Dans le tampon PCRx3, avec de hautes concentrations de MgCI2 (4.5 mM) et de dNTPs (750 µM), la conséquence de la température est moins critique, et nous avons obtenu les meilleurs résultats à une température uniforme (figure 12S : 78/74-75 et figure 12M : 88/84-85). Cependant, le meilleur ratio signal spécifique/bruit de fond a été obtenu avec l'expérience en une étape (figure 12M/K7 et figure 12S/K8) grâce à des conditions de température élevée pour la transcription inverse.

### EXEMPLE 10 : influence de la concentration en MgCl₂dans le mélange réactionnel sur l'efficience et la sensibilité de la méthode en une étape

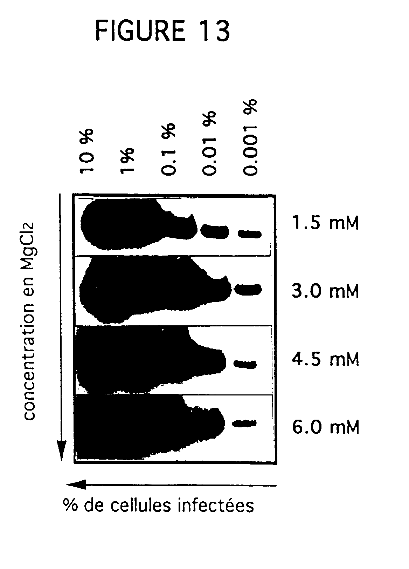

Les conditions expérimentales utilisées sont essentiellement identiques à celles décrites dans l'exemple 1. L'ARN est extrait de cellules H9 infectées par la souche HTLV-IIIB, les cellules infectées représentant de 10 % à 0,001 % de la population traitée. Les amorces d'amplification sont 1082-972 (voir tableau 1 et figure 1). La concentration finale du tampon utilisé est 10 mM Tris pH 8.3, 50 mM KCI, MgCl₂ variant de 1.5 mM à 6 mM, 0,01 % gélatine. Les concentrations finales sont : amplimers 310 nM, dNTPs 250 µM, Taq polymérase (Perkin Elmer-Cetus) 2,5 U, RT-AMV (Boehringer-Mannheim) 10U. Le protocole d'amplification, réalisé dans un thermo-cycleur, consiste en une incubation de 10 minutes à 65°C (dénaturation de l'ARN et hybridation du primer inverse lui-même identique à l'amplimer 3'), puis 8 minutes à 50°C (transcription réverse), puis 5 minutes à 95°C (dénaturation des hybrides ADN/ARN et inactivation de la transcriptase réverse), puis consécutivement, 35 cycles de PCR sont alors réalisés, un cycle consistant en 1 minute à 95°C pour la dénaturation de l'ADN, 1 minute à 55°C pour l'hybridation des amplimers sur les matrices ADN monocaténaires, et 1 minute à 72°C pour l'élongation des amorces oligonucléotidiques (amplimers) ; le dernier cycle présente de plus une extension de 7 minutes de l'étape d'élongation afin de terminer la synthèse des fragments d'ADN incomplets. Les produits de RT-PCR sont ensuite analysés en southern blot. L'analyse en southern blot d'ARNs amplifiés par les primers 1082-972 (voir tableau 1 et figure 1) met en évidence en autoradiographie la bande de 340 nucléotides attendue (figure 13), révélée avec la sonde 338 (tableau 1, figure 1). La concentration en MgCl₂ n'influence de façon notable ni la sensibilité ni l'efficience de la méthode. Dans tous les cas, on peut détecter une proportion aussi faible qu'une cellule infectée sur 100.000, et le signal généré par la méthode est corrélé à la concentration initiale d'ARN-cible.

### EXEMPLE 11: influence du ratio RT AMV-Taq polymérase dans le mélange réactionnel sur l'efficience et la sensibilité de la méthode en une étape

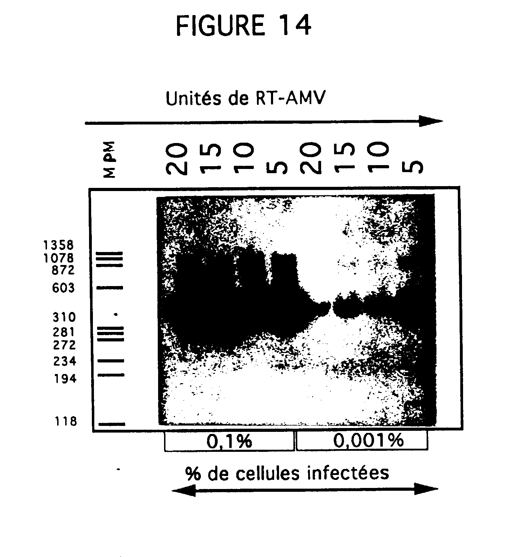

Les conditions expérimentales utilisées sont essentiellement identiques à celles décrites dans l'exemple 1. L'ARN est extrait de cellules H9 infectées par la souche HTLV-IIIB, les cellules infectées représentant de 0,001 % ou 0,1 % de la population traitée. Les primers d'amplification sont 1082-972 (voir tableau 1 et figure 1). La concentration finale du tampon utilisé est 10mM Tris pH 8,3, 50 mM KCl, 1,5 mM MgCl₂, 0,01 % gélatine. Les concentrations finales sont : amplimers 310 nM, dNTPs 250 µM, Taq polymérase (Perkin Elmer-Cétus) 2.5 U, RT-AMV (Boehringer-Mannheim) 5 U, 10 U, 15 U ou 20 U. Le protocole d'amplification, réalisé dans un thermo-cycleur, consiste en une incubation de 10 minutes à 65°C (dénaturation de l'ARN et hybridation du primer inverse lui-même identique à l'amplimer 3'), puis 8 minutes à 50°C (transcription réverse), puis 5 minutes à 95°C (dénaturation des hybrides ADN/ARN et inactivation de la transcriptase réverse), puis consécutivement, 35 cycles de PCR sont alors réalisés, un cycle consistant en 1 minute à 95°C pour la dénaturation de l'ADN, 1 minute à 55°C pour l'hybridation des amplimers sur les matrices ADN monocaténaires, et 1 minute à 72°C pour l'élongation des amorces oligonucléotidiques (amplimers) ; le dernier cycle présente de plus une extension de 7 minutes de l'étape d'élongation afin de terminer la synthèse des fragments d'ADN incomplets. Les produits de RT-PCR sont ensuite analysés en southern blot. L'analyse en southern blot d'ARNs amplifiés par les primers 1082-972 (voir tableau 1 et figure 1) met en évidence en autoradiographie la bande de 340 nucléotides attendue (figure 14), révélée avec la sonde 338 (tableau 1, figure 1). Les variations du ratio RT AMV-Taq polymérase n'influencent de façon notable ni la sensibilité ni l'efficience de la méthode. Dans tous les cas, il est possible de détecter une proportion aussi faible qu'une cellule infectée sur 100.000 et le signal généré par la méthode est corrélé à la concentration initiale d'ARN cible. On observe cependant une baisse sensible du signal avec un ratio égal à 8.

## Revendications

1. Procédé d'amplification d'au moins une séquence spécifique d'ARN, contenu ou susceptible d'être contenu dans un échantillon, ledit ARN contenant ou étant susceptible de contenir des structures secondaires, comprenant les étapes consistant à :
- obtenir une solution de départ contenant l'ARN dudit échantillon dans un tampon approprié,
- dénaturer par chauffage l'ARN contenu dans ladite solution,
- traiter la solution obtenue, contenant l'ARN dénaturé, avec une première amorce oligonucléotidique capable de s'hybrider avec ledit ARN au voisinage de l'extrémité 3' de ladite séquence spécifique, dans des conditions permettant l'hybridation de ladite première amorce suivie de la synthèse d'un premier brin d'ADN complémentaire (ADNc) de la séquence d'ARN à amplifier, en présence d'une quantité suffisante d'un système enzymatique ayant, dans ledit tampon, une activité de transcriptase réverse,
- dénaturer l'hétéroduplex ARN-ADNc formé et traiter la solution obtenue avec une seconde amorce oligonucléotidique capable de s'hybrider avec une séquence située au voisinage de l'extrémité 3' dudit premier brin d'ADN complémentaire, dans des conditions permettant l'hybridation de ladite seconde amorce suivie de la synthèse du second brin d'ADNc, en présence d'un second système enzymatique, thermostable, ayant, dans le même tampon, une activité d'ADN polymérase,
- et à soumettre l'ADNc obtenu à des cycles d'amplification en nombre suffisantpour obtenir le degré d'amplification désiré,
caractérisé par le fait que ladite solution contient dès le départ tous les réactifs et solvants nécessaires auxdites étapes, que l'ensemble des étapes est effectué dans un même conteneur et sans ajout de réactifs ou de solvants, que l'on met en oeuvre ledit chauffage dans des conditions de température et de durée suffisantes pour effectuer ladite dénaturation et que ledit système enzymatique ayant une activité de transcriptase réverse est un système enzymatique sélectionné capable de supporter lesdites conditions de température et de durée.

2. Procédé selon la revendication 1, caractérisé par le fait que l'on effectue la dénaturation de l'ARN par chauffage à une température supérieure ou égale à 60°C, en particulier comprise entre 60 et 75°C, et notamment au moins égale à 65°C.

3. Procédé selon la revendication 2, caractérisé par le fait que l'on effectue ladite dénaturation pendant 1 à 15 minutes.

4. Procédé selon la revendication 2 ou 3, caractérisé par le fait que ladite première amorce a une longueur suffisante pour pouvoir s'hybrider à l'ARN à la température de dénaturation choisie.

5. Procédé selon l'une quelconque des revendications 2 à 4, caractérisé par le fait que l'on effectue la dénaturation de l'ARN et l'hybridation pendant un temps suffisant pour permettre l'hybridation de la première amorce, mais non supérieur à une durée prédéterminée au-delà de laquelle le système enzymatique à activité de transcriptase réverse serait inactivé.

6. Procédé selon l'une quelconque des revendications précédentes, caractérisé par le fait qu'après l'étape de dénaturation et d'hybridation, on porte la solution jusqu'à une température prédéterminée choisie pour l'étape de transcription réverse, ladite température étant suffisamment élevée pour permettre le maintien de l'hybridation de la première amorce dans des conditions suffisamment discriminantes, et étant au moins égale à 45°C.

7. Procédé selon la revendication précédente, caractérisé par le fait que ladite température prédéterminée est au moins égale à 50°C.

8. Procédé selon l'une quelconque des revendications 1 à 5, caractérisé par le fait qu'après l'étape de dénaturation de l'ARN, on refroidit la solution à une température suffisamment basse pour permettre l'hybridation de l'amorce à une séquence d'ARN non rigoureusement complémentaire, pendant un temps suffisamment faible pour éviter des appariements non spécifiques, puis on réchauffe la solution jusqu'à une température prédéterminée choisie pour l'étape de transcription réverse et au moins égale à 45°C.

9. Procédé selon la revendication 8, caractérisé par le fait que ladite température suffisamment basse est au moins égale à 40°C.

10. Procédé selon l'une quelconque des revendications 6 à 9, caractérisé par le fait qu'on laisse agir le système ayant l'activité transcriptase réverse à ladite température prédéterminée pendant un temps permettant d'obtenir un brin d'ADNc ayant une longueur suffisante pour contenir au moins la séquence complémentaire de la deuxième amorce.

11. Procédé selon l'une quelconque des revendications précédentes, caractérisé par le fait que l'on effectue l'étape de transcription réverse à une température comprise entre 50 et 75°C environ, et en particulier entre 50 et 65°C environ.

12. Procédé selon l'une quelconque des revendications précédentes, caractérisé par le fait que la durée de l'étape de transcription réverse est inférieure à 15 minutes, et en particulier peut varier de 1 à 10 minutes environ.

13. Procédé selon l'une quelconque des revendications 1 à 11, caractérisé par le fait que l'on utilise une transcriptase réverse non réputée thermostable.

14. Procédé selon la revendication précédente, caractérisé par le fait que ladite transcriptase réverse est choisie parmi celles de virus de la myéloblastose aviaire (RT-AMV) et celles de virus de Maloney de la leucémie murine.

15. Procédé selon l'une quelconque des revendications précédentes, caractérisé par le fait que l'on utilise des quantités de transcriptase réverse et d'ADN polymérase telles que leur rapport, exprimé en unités de RT-AMV et de Taq polymérase, respectivement, est au moins égal à 2 et inférieur à 8, et peut varier notamment de 2 à 6, ou que, lorsqu'on choisit une transcriptase réverse et/ou une ADN polymérase autres que la RT-AMV et la Taq polymérase, respectivement, on utilise ces enzymes en quantités telles que des quantités équivalentes de RT-AMV et de Taq polymérase, respectivement, soient dans le même rapport au moins égal à 2 et inférieur à 8.

16. Procédé selon l'une quelconque des revendications précédentes, caractérisé par le fait que l'on dénature l'hétéroduplex par chauffage à une température suffisante, et en particulier à une température, supérieure à 90°C, pouvant être supportée, sans inactivation notable, par l'ADN polymérase.

17. Procédé selon l'une quelconque des revendications précédentes, caractérisé par le fait que l'on effectue l'étape d'hybridation de la seconde amorce à une température permettant l'hybridation dans des conditions suffisamment discriminantes.

18. Procédé selon l'une quelconque des revendications 1 à 16, caractérisé par le fait qu'après l'étape de dénaturation de l'hétéroduplex, on refroidit la solution à une température suffisamment basse pour permettre l'hybridation de la seconde amorce à une séquence d'ADN non rigoureusement complémentaire, pendant un temps suffisamment faible pour éviter des appariements non spécifiques, puis on réchauffe la solution jusqu'à une température prédéterminée choisie pour l'étape de synthèse du second brin d'ADNc.

19. Procédé selon l'une quelconque des revendications 17 et 18, caractérisé par le fait que l'on effectue la synthèse du second brin d'ADNc à une température suffisamment élevée compatible avec le maintien de l'hybridation de la seconde amorce.

20. Procédé selon l'une quelconque des revendications précédentes, caractérisé par le fait qu'après la synthèse du second brin d'ADNc, on soumet l'ADNc double-brin obtenu à un nombre suffisant de cycles d'amplification par dénaturation de l'ADN double-brin, hybridation des amorces sur les brins d'ADN néoformés dont elles sont respectivement complémentaires, et synthèse de produits d'élongation sous l'action de l'ADN polymérase.

21. Procédé selon l'une quelconque des revendications précédentes, caractérisé par le fait que l'on opère *in situ* sur coupes de tissus, frottis ou autres prélèvements déposés ou fixés sur un support.

22. Procédé selon l'une quelconque des revendications précédentes, pour l'amplification d'ARN du virus HIV1, caractérisé par le fait que la première amorce comprend la séquence suivante :

CCTATCTGTCCCCTCAGCTAC

23. Procédé selon l'une quelconque des revendications précédentes, pour l'amplification d'ARN du virus HIV1, caractérisé par le fait que la seconde amorce comprend la séquence suivante :

TCTATCAAAGCAACCCAC

## Claims

1. Method for the amplification of at least one specific sequence of RNA contained or likely to be contained in a sample, the said RNA containing or likely to contain secondary structures, comprising the steps consisting in:
- obtaining a starting solution containing the RNA of the said sample in a suitable buffer,
- denaturing the RNA contained in the said solution by heating,
- treating the solution obtained, containing the denatured RNA, with a first oligonucleotide primer capable of hybridizing with the said RNA in the vicinity of the 3' end of the said specific sequence, under conditions permitting hybridization of the said first primer followed by the synthesis of a first DNA strand complementary (cDNA) to the RNA sequence to be amplified, in the presence of a sufficient quantity of an enzyme system having reverse transcriptase activity in the said buffer,
- denaturing the RNA-cDNA heteroduplex formed, and treating the solution obtained with a second oligonucleotide primer capable of hybridizing with a sequence situated in the vicinity of the 3' end of the said first complementary DNA strand, under conditions permitting hybridization of the said second primer followed by the synthesis of the second cDNA strand, in the presence of a second, heat-stable enzyme system having DNA polymerase activity in the same buffer,
- and in subjecting the cDNA obtained to a sufficient number of amplification cycles to obtain the desired degree of amplification,
characterized in that the said solution contains, right from the start, all the reactants and solvents needed for the said steps, in that all the steps are performed in the same container and without addition of reactants or solvents, in that the said heating is employed under conditions of temperature and duration which are sufficient to effect the said denaturation and in that the said enzyme system having reverse transcriptase activity is a selected enzyme system which is capable of withstanding the said conditions of temperature and duration.

2. Method according to Claim 1, characterized in that the denaturation of the RNA is performed by heating to a temperature above or equal to 60°C, especially of between 60 and 75°C and in particular equal to at least 65°C.

3. Method according to Claim 2, characterized in that the said denaturation is performed for 1 to 15 minutes.

4. Method according to Claim 2 or 3, characterized in that the said first primer has a sufficient length to be capable of hybridizing with the RNA at the chosen denaturation temperature.

5. Method according to any one of Claims 2 to 4, characterized in that the denaturation of the RNA and the hybridization are performed for a sufficient time to permit hybridization of the first primer, but not longer than a predetermined period beyond which the enzyme system having reverse transcriptase activity would be inactivated.

6. Method according to any one of the preceding claims, characterized in that, after the denaturation and hybridization step, the solution is brought to a predetermined temperature chosen for the reverse transcription step, the said temperature being sufficiently high to permit maintenance of the hybridization of the first primer under sufficiently discriminating conditions, and being at least equal to 45°C.

7. Method according to the preceding claim, characterized in that the said predetermined temperature is equal to at least 50°C.

8. Method according to any one of Claims 1 to 5, characterized in that, after the RNA denaturation step, the solution is cooled to a sufficiently low temperature to permit hybridization of the primer to an RNA sequence which is not absolutely complementary, for a sufficiently short time to prevent non-specific pairings, and the solution is then heated to a predetermined temperature chosen for the reverse transcription step and at least equal to 45°C.

9. Method according to Claim 8, characterized in that the said sufficiently low temperature is equal to at least 40°C.

10. Method according to any one of Claims 6 to 9, characterized in that the system having reverse transcriptase activity is allowed to act at the said predetermined temperature for a time which makes it possible to obtain a cDNA strand having a sufficient length to contain at least the sequence complementary to the second primer.

11. Method according to any one of the preceding claims, characterized in that the reverse transcription step is performed at a temperature of between 50 and 75°C approximately, and especially between 50 and 65°C approximately.

12. Method according to any one of the preceding claims, characterized in that the duration of the reverse transcription step is less than 15 minutes and, in particular, can vary from 1 to 10 minutes approximately.

13. Method according to any one of Claims 1 to 11, characterized in that a reverse transcriptase which is not renowned for its heat stability is used.

14. Method according to the preceding claim, characterized in that the said reverse transcriptase is chosen from those of the avian myeloblastosis virus (RT-AMV) and those of the Moloney murine leukaemia virus.

15. Method according to any one of the preceding claims, characterized in that quantities of reverse transcriptase and of DNA polymerase are used such that their ratio, expressed in RT-AMV and Taq polymerase units, respectively, is equal to at least 2, and less than 8, and can vary, in particular from 2 to 6, or in that, when a reverse transcriptase and/or a DNA polymerase other than RT-AMV and Taq polymerase, respectively, is/are chosen, these enzymes are used in quantities such that equivalent quantities of RT-AMV and of Taq polymerase, respectively, are in the same ratio equal to at least 2 and less than 8.

16. Method according to any one of the preceding claims, characterized in that the heteroduplex is denatured by heating to a sufficient temperature, and especially to a temperature, above 90°C, capable of being withstood by the DNA polymerase without appreciable inactivation.

17. Method according to any one of the preceding claims, characterized in that the step of hybridization of the second primer is performed at a temperature permitting hybridization under sufficiently discriminating conditions.

18. Method according to any one of Claims 1 to 16, characterized in that, after the step of denaturation of the heteroduplex, the solution is cooled to a sufficiently low temperature to permit hybridization of the second primer to a DNA sequence which is not absolutely complementary, for a sufficiently short time to prevent non-specific pairings, and the solution is then heated to a predetermined temperature chosen for the step of synthesis of the second cDNA strand.

19. Method according to either of Claims 17 and 18, characterized in that the synthesis of the second cDNA strand is performed at a sufficiently high temperature compatible with maintenance of the hybridization of the second primer.

20. Method according to any one of the preceding claims, characterized in that, after the synthesis of the second cDNA strand, the double-stranded cDNA obtained is subjected to a sufficient number of cycles of amplification by denaturation of the double-stranded DNA, hybridization of the primers with the newly formed DNA strands to which they are respectively complementary and synthesis of elongation products under the action of the DNA polymerase.

21. Method according to any one of the preceding claims, characterized in that the procedure is carried out *in situ* on tissue sections, smears or other samples deposited on or attached to a support.

22. Method according to any one of the preceding claims, for the amplification of HIV-1 virus RNA, characterized in that the first primer comprises the following sequence:

CCTATCTGTCCCCTCAGCTAC.

23. Method according to any one of the preceding claims, for the amplification of HIV-1 virus RNA, characterized in that the second primer comprises the following sequence:

TCTATCAAAGCAACCCAC

## Patentansprüche

1. Verfahren zur Amplifikation mindestens einer spezifischen RNS-Sequenz, die eine Sonde enthält oder diese enthalten kann, wobei die RNS Sekundärstrukturen aufweist oder diese aufweisen kann, welches die folgenden Schritte umfaßt:
- Erhalten einer Stammlösung, die die RNS der Sonde in einem geeigneten Puffer enthält,
- Denaturieren der in dieser Lösung enthaltenen RNS mittels Erhitzen,
- Behandeln der erhaltenen Lösung, die die denaturierte RNS enthält, mit einem ersten Oligonukleotidprimer, der mit der besagten RNS in der Nähe des 3'-Endes der besagten spezifischen Sequenz hybridisieren kann, und zwar unter Bedingungen, welche die Hybridisierung des ersten Primers gefolgt von der Synthese eines ersten komplementären DNS-Strangs (cDNS) und Amplifizieren der Sequenz der RNS in Anwesenheit einer ausreichenden Menge eines Enzymsystems mit Reversetranskriptase-Aktivität in dem Puffer,
- Denaturieren der gebildeten RNS-cDNS-Heteroduplex und Behandeln der erhaltenen Lösung mit einem zweiten Oligonukleotidprimer, der mit einer Sequenz in der Nähe des 3'-Endes des ersten komplementären DNS-Strangs hybridisieren kann, und zwar unter Bedingungen, die die Hybridisierung des zweiten Primers gestatten, gefolgt von der Synthese eines zweiten cDNS-Strangs in Anwesenheit eines zweiten, thermostabilen Enzymsystems mit DNS-Polymerase-Aktivität im selben Puffer, und
- Unterwerfen der erhaltenen cDNS unter Amplifikationszyklen in ausreichender Anzahl zum Erhalt des gewünschten Amplifikationsgrades,
dadurch gekennzeichnet, daß die besagte Lösung von Anfang an alle für die besagten Schritte notwendigen Reagentien und Lösungsmittel enthält, daß die Schrittsequenz in ein und demselben Behältnis und ohne Zugabe von Reagentien oder Lösungsmitteln bewirkt wird, daß das Erwärmen unter Temperaturbedingungen und für ausreichende Zeit zum Erzielen der Denaturierung erfolgt, und daß das Enzymsystem mit Reversetranskriptase-Aktivität ein Enzymsystem ist, das ausgewählt ist nach seiner Fähigkeit, die besagten Temperaturbedingungen und Zeiten auszuhalten.

2. Verfahren gemäß Anspruch 1, dadurch gekennzeichnet, daß man die Denaturierung der RNS durch Aufheizen auf eine Temperatur oberhalb oder gleich 60 °C, insbesondere zwischen 60 °C und 75 °C und noch spezieller gleich 65 °C, bewirkt.

3. Verfahren gemäß Anspruch 2, dadurch gekennzeichnet, daß man die Denaturierung in einer Zeitspanne von 1 bis 15 Minuten bewirkt.

4. Verfahren gemäß Anspruch 2 oder 3, dadurch gekennzeichnet, daß der besagte erste Primer zum Hybridisieren mit der RNS bei der gewählten Denaturierungsteperatur ausreichende Länge aufweist.

5. Verfahren gemäß einem der Ansprüche 2 bis 4, dadurch gekennzeichnet, daß man die Denaturierung der RNS und die Hybridisierung über eine zum Hybridisieren des ersten Primers ausreichende Zeit, aber nicht über eine bestimmte Zeit hinaus, bewirkt,nach der das Enzymsystem mit Reversetranskriptase-Aktivität inaktiviert wird.

6. Verfahren gemäß einem der vorstehenden Ansprüche, dadurch gekennzeichnet, daß man nach dem Schritt der Denaturierung und Hybridisierung die Lösung auf eine vorbestimmte, für den Schritt der reversen Transkription gewählte Temperatur bringt, wobei die Temperatur ausreichend hoch ist, um die Hybridisierung des ersten Primers unter ausreichend unterschiedlichen Bedingungen aufrechterhalten zu können, und wobei die Temperatur mindestens 45 °C beträgt.

7. Verfahren gemäß dem vorstehenden Anspruch, dadurch gekennzeichnet, daß die vorbestimmte Temperatur mindestens 50 °C beträgt.

8. Verfahren gemäß einem der Ansprüche 1 bis 5, dadurch gekennzeichnet, daß man nach dem Denaturieren der RNS die Lösung auf eine ausreichend tiefe Temperatur kühlt, um die Hybridisierung des Primers an eine nicht strikt komplementäre RNS-Sequenz zu gestatten, für eine zum Erzielen unspezifischer Paarungen ausreichend förderliche Zeit auf dieser Temperatur hält und anschließend die Lösung auf eine vorbestimmte Temperatur, die für den Schritt der reversen Transkriptions gewählt wurde und die mindestens 45 °C beträgt, erwärmt.

9. Verfahren gemäß Anspruch 8, dadurch gekennzeichnet, daß die ausreichend tiefe Temperatur mindestens 40 °C beträgt.

10. Verfahren gemäß einem der Ansprüche 6 bis 9, dadurch gekennzeichnet, daß man das System mit Reversetranskriptase-Aktivität bei der vorbestimmten Temperatur für eine Zeit reagieren läßt, die den Erhalt eines cDNS-Strangs mit ausreichender Länge gestattet, so daß dieser mindestens eine dem zweiten Primer komplementäre Sequenz enthalten kann.

11. Verfahren gemäß einem der vorstehenden Ansprüche, dadurch gekennzeichnet, daß man den Schritt der reversen Transkription bei einer Temperatur zwischen etwa 50 °C und 75 °C und insbesondere zwischen etwa 50 °C und 65 °C bewirkt.

12. Verfahren gemäß einem der vorstehenden Ansprüche, dadurch gekennzeichnet, daß die Dauer des Transkriptionsschritts unter 15 Minuten liegt und insbesondere zwischen etwa 1 und 10 Minuten schwanken kann.

13. Verfahren gemäß einem der Ansprüche 1 bis 11, dadurch gekennzeichnet, daß man eine nicht als thermostabil betrachtete Reversetranskriptase verwendet.

14. Verfahren gemäß dem vorstehenden Anspruch, dadurch gekennzeichnet, daß die besagte Reversetranskriptase unter denjenigen des Vogel-Myeloblastose-Virus (RT-AMV = RT-aviaire myéloblastose virus) und denjenigen des Maloney-Leukämievirus der Maus ausgewählt ist.

15. Verfahren gemäß einem der vorstehenden Ansprüche, dadurch gekennzeichnet, daß man Mengen an Reversetranskriptase und DNS-Polymerase in einem Verhältnis, ausgedrückt in RT-AMV-Einheiten bzw. Einheiten der Taq-Polymerase von mindestens gleich 2 und unter 8, das insbesondere zwischen 2 und 6 schwanken kann, einsetzt, und daß man dann, wenn eine andere Reversetranskriptase und/oder DNS-Polymerase als die AMV-Reversetranskriptase bzw. Taq-Polymerase gewählt wird, diese Enzyme in Mengen verwendet werden, die den Mengen an AMV-Reversetranskriptase und Taq-Polymerase äquivalent sind, damit sie in demselben Verhältnis von mindestens gleich 2 und unter 8 liegen.

16. Verfahren gemäß einem der vorstehenden Ansprüche, dadurch gekennzeichnet, daß man die Heteroduplex durch Aufheizen auf ausreichende Temperatur denaturiert und insbesondere auf eine Temperatur über 90 °C, welche die DNS-Polymerase ohne merkliche Inaktivierung aushält.

17. Verfahren gemäß einem der vorstehenden Ansprüche, dadurch gekennzeichnet, daß man den Hybridisierungsschritt des zweiten Primers bei einer Temperatur bewirkt, die die Hybridisierung unter ausreichend unterschiedlichen Bedingungen gestattet.

18. Verfahren gemäß einem der Ansprüche 1 bis 16, dadurch gekennzeichnet, daß man nach dem Schritt der Denaturierung der Heteroduplex die Lösung auf ausreichend tiefe Temperatur abkühlt, um die Hybridisierung des zweiten Primers mit einer nicht strikt komplementären DNS-Sequenz zu gestatten, bei zum Bewirken unspezifischer Paarungen ausreichend förderlicher Temperatur hält und anschließend die Lösung auf eine vorbestimmte Temperatur erwärmt, die für den Syntheseschritt des zweiten cDNS-Strangs gewählt wurde.

19. Verfahren gemäß einem der Ansprüche 17 und 18, dadurch gekennzeichnet, daß man die Synthese des zweiten cDNS-Strangs bei ausreichend hoher Temperatur bewirkt, die mit dem Beibehalten der Hybridisierung des zweiten Primers kompatibel ist.

20. Verfahren gemäß einem der vorstehenden Ansprüche, dadurch gekennzeichnet, daß man nach der Synthese des zweiten cDNS-Strangs den erhaltenen cDNS-Doppelstrang einer ausreichenden Zahl von Amplifikationszyklen unterwirft, indem man den DNS-Doppelstrang denaturiert, die Primer mit den neu gebildeten DNS-Strängen hybridisiert, denen sie jeweils komplementär sind, und die Elongationsprodukte durch die DNS-Polymerase-Aktivität synthetisiert.

21. Verfahren gemäß einem der vorstehenden Ansprüche, dadurch gekennzeichnet, daß man in situ auf Abschnitten von Gewebe, Abrieben oder anderen Proben, die auf einem Träger abgeschieden oder befestigt sind, arbeitet.

22. Verfahren gemäß einem der vorstehenden Ansprüche zur Amplifikation von RNS eines HIV1-Virus, dadurch gekennzeichnet, daß der erste Primer die folgende Sequenz aufweist:

CCTATCTGTCCCCTCAGCTAC

23. Verfahren gemäß einem der vorstehenden Ansprüche zur Amplifikation der RNS des HIV1-Virus, dadurch gekennzeichnet, daß der zweite Primer die folgende Sequenz aufweist:
